# EUROPEAN PATENT SPECIFICATION

(11) **EP 1 891 433 B1**
(45) Date of publication and mention of the grant of the patent: **07.03.2012**
(21) Application number: 06773407.9
(22) Date of filing: 16.06.2006
(51) Int. Cl.: G01N 33/48, G01N 31/00, G06G 7/48

(54) **METHOD OF CLASSIFYING CHEMICALLY CROSSLINKED CELLULAR SAMPLES USING MASS SPECTRA**
VERFAHREN ZUR KLASSIFIZIERUNG CHEMISCH QUERVERNETZTER ZELLULÄRER PROBEN UNTER VERWENDUNG VON MASSENSPEKTREN
PROCÉDÉ DE CLASSIFICATION D'ÉCHANTILLONS CELLULAIRES CHIMIQUEMENT RÉTICULÉS PAR SPECTRE DE MASSE

(30) Priority: 16.06.2005 US 691182 P
(43) Date of publication of application: 27.02.2008
(73) Proprietor: 3M Innovative Properties Company, St. Paul, MN 55133-3427 (US)
(72) Inventor: CHONG CONKLIN, Bathsheba E., Saint Paul, MN 55133-3427 (US); PARKS, Patrick J., Saint Paul, MN 55133-3427 (US)
(74) Representative: Vossius & Partner
(86) International application number: PCT/US2006/023594
(87) International publication number: WO 2006/138629

(56) References cited:
- WO-A-03/031031
- WO-A-2006/127860
- WO-A-2006/127861
- US-B1- 6 897 072
- SHI SHAN-RONG ET AL: "ANTIGEN RETRIEVAL TECHNIQUE UTILIZING CITRATE BUFFER OR UREA SOLUTION FOR IMMUNOHISTOCHEMICAL DEMONSTRATION OF ANDROGEN RECEPTOR IN FORMALIN-FIXED PARAFFIN SECTIONS" JOURNAL OF HISTOCHEMISTRY AND CYTOCHEMISTRY, HISTOCHEMICAL SOCIETY, NEW YORK, NY, US, vol. 41, no. 11, 1 January 1993 (1993-01-01), pages 1599-1604, XP009046607 ISSN: 0022-1554

## Description

Microscopic examination and histopathologic diagnosis of both human and animal tissues has aided in the accuracy of medical diagnosis and treatment, as well as the advancement of research into diseases and their potential treatments. Advances in analytical techniques have provided the opportunity to understand the cellular mechanisms of disease and to select appropriate treatments. The identification of molecular markers of disease, such as tumor-specific antigens, has enabled diagnostic and prognostic assays to be developed that rely on the use of molecular probes (e.g., antibodies and nucleic acid probes) to detect these markers.

Identifying novel markers is one of the earliest and most difficult steps in the diagnostics and drug discovery processes. One way to discover if substances are markers for a disease is by determining if they are "differentially expressed" in biological samples from patients exhibiting the disease as compared to samples from patients not having the disease. For example, in mass spectra of samples comparing a group of diseased patients and normal patients, the average intensity of the generated signals at the mass-to-charge ratio A is higher in the samples from diseased patients than the samples from the normal patients. The marker at the mass-to-charge ratio A is said to be "differentially expressed" in diseased patients, because the concentration of this marker is, on average, greater in samples from diseased patients than in samples from normal patients. Since the concentration of the marker is generally greater in samples from diseased patients than in the normal samples, the marker can also be characterized as being "up-regulated" for the disease. If the concentration of the marker was generally less in the samples from diseased patients than in the samples from normal patients, the protein could be characterized as being "down-regulated".

Once markers are discovered, they can be used as diagnostic tools. For example, with reference to the example described above, an unknown sample from a test patient may be analyzed using a mass spectrometer and a mass spectrum can be generated. The mass spectrum can be analyzed and the intensity of a signal at the mass-to-charge ratio A can be determined in the test patient's mass spectrum. The signal intensity can be compared to the average signal intensities at the mass-to-charge ratio A for diseased patients and normal patients. A prediction can then be made as to whether the unknown sample indicates that the test patient has or will develop cancer. For example, if the signal intensity at the mass-to-charge ratio A in the unknown sample is much closer to the average signal intensity at the mass-to-charge ratio A for the diseased patient spectra than for the normal patient spectra, then a prediction can be made that the test patient is more likely than not to develop or have the disease.

When a large number of mass spectra of a large number of biological samples are analyzed, it is not readily apparent which signals represent markers that might differentiate between a diseased state and a non-diseased state. A typical mass spectrum of a biological sample has numerous potential marker signals (e.g., greater than 200) and a significant amount of noise. This can make the identification of potentially significant signals and the identification of average signal differentials difficult. Consequently, it is difficult to identify and quantify potential markers. Unless the potential markers exhibit strong up-regulation or strong down-regulation, the average signal differential between samples from diseased patients and samples from normal patients may not be easily discernable.

While the described differential expression analysis is useful, the samples used to generate the mass spectra have excluded formalin-fixed paraffin-embedded tissue. DNA has been isolated from paraffin embedded tissue specimens following chemical fixation, typically with formalin. However, the methods involved in the formation of paraffin sections have heretofore excluded these sections from most of the molecular analytic methods, including mass spectrometry.

Historically, formalin fixation has been used with tissue in order to provide optimal specimen preservation for light microscopic examination of the preserved tissue. Chemical fixation with aldehydes is associated with denaturation that results from the crosslinking of pendant reactive amines. Formalin fixation results in methylene bridges between and among proteins, effectively reducing or removing the tertiary structure required for immune detection of proteins. Further, paraffin embedding is carried out at temperatures that can cause the loss of tertiary structure of the proteins thereby forming unfolded, but intact, proteins, reducing or removing enzymatic activity where it exists as well as removing, the structures (epitopes) required for immune detection.

Standard histological staining methods such as haematoxylin and eosin (H&E) generally can reveal only a limited amount of information. Current methods of microscopic evaluation can be extended to include such methods as morphometry, immunohistochemistry, in situ hybridization, etc. The identification and development of new clinically important molecular markers has been impeded by the slow and tedious process of determining the expression of these markers in large numbers of clinical specimens.

The natural progression of the data from the human genome project has been from single gene to multiple genes (genomics) and subsequently to identifying all proteins (proteomics) simultaneously. While "protein chips" carry the potential to measure concentrations, and perhaps function, at present immunohistochemistry is the only method capable of localization. Localization by immunohistochemistry is qualitative by nature, and semiquantitative at best using subjective evaluation by trained evaluators.

The ability to identify potential drug targets for potential treatment using immunohistochemistry has been amplified by the use of tissue microarrays (TMAs), a technology that involves the placement of many, typically 500 to 1000, tissue samples on a single microscope slide. Methods of grouping multiple tissue specimens on a single substrate have relied on manually cutting multiple paraffin-embedded tissue specimens and forming them into a composite block (see, e.g., Battifora et al., 1986, Lab. Invest. 55: 244-248; U.S. Pat. No. 4,820,504) or into "straws" or "logs" from which transverse sections could be obtained (see, e.g., Wan et al., 1987, J. Immunol. Meth. 103: 121-129; U.S. Pat. No. 4,914,022; Miller and Groothuis, 1991, A.J.C.P. 96: 228-232); and Kononen et al., 1998, Nat. Med. 4: 844-7, which describes a technique for generating tissue arrays comprising hundreds of tumor specimens using punched samples from archival tissue blocks.

Tissue microarrays have the capacity to measure insoluble, large proteins such as extracellular matrix proteins, currently unavailable for analysis with standard mass spectrometric methods. Additionally, tissue microarrays complement protein microarrays, which have the potential to measure soluble proteins. However, a major difficulty with TMAs is the limited amount of data that comes with each "histospot" (the 0.15 cm diameter tissue section spotted onto the microarray).

Tissue Microarrays (TMAs) represent the tissue equivalent of DNA arrays and protein arrays. The concept of having more than one tissue on a glass slide is old, but the idea of arranging specific samples in a fixed array by using subsets of already existing tissues only was described in 1998 *[*Kononen, J.; Bubendorf, L.; Kallioniemi, A.; Barlund, M.; Schraml, P.; Leighton, S.; Torhorst, J.; Mihatsch, M.J.; Sauter, G.; Kallioniemi, O.P. Tissue microarrays for high-throughput molecular profiling of tumor specimens. Nat. Med 1998, 4: 844-847*.]*. Most of the TMAs used to date have been malignant tumors, in part because they represent a major reservoir of tissue since all patients with putative tumors must have a tissue diagnosis in order to begin therapy and in part because the problems of cancer treatment have been so intractable. Approximately 2-4% of all patients (∼50 000) in the United States with cancer fail to have a primary tumor identified. These patients receive therapy for their cancers on an empirical basis. The ability to use their tissue samples taken for the diagnosis of malignancy in order to identify the origin of the malignancy will determine their therapy.

WO 2004/080579 A2 describes liquid tissue preparation from histologically processed biological samples, tissues and cells which are directly converted into a multi-use biomolecule lysate and can be analyzed by MALDI mass spectroscopy.

The article of Shi Shan-Rong et al. in The Journal of Histochemistry and Cytochemistry, Vol. 41, No. 11, pp. 1599-1604, 1993 describes antigen retrieval technique utilizing citrate buffer or urea solution for immunohistochemical demonstration of androgen receptor in formalin-fixed paraffin sections including a protocol for pre-treatment before the immunostaining procedure.

WO 03/031031 A1 describes a method for analyzing mass spectra using a digital computer wherein each sample from plurality of samples is assigned to a class within a class set having two or more classes and each class is characterized by a different biological status. A classification model discriminates between classes in the class set.

The invention is generally to the analysis of cellular samples (e.g., cells, tissues, organs) that include a chemically crosslinked analyte (e.g., formalin-fixed proteins), wherein the sample is embedded in an organic solid material (e.g., paraffin) involving the use of mass spectrometry. In particular, the invention refers to a computerized method of analyzing an analyte according to claim 1.

More specifically, the present invention provides a method of analysis using mass spectrometry of chemically fixed, paraffin-embedded, tissues following reversal of at least a portion of the chemically crosslinked analytes (e.g., crosslinked proteins) to form decrosslinked analytes in a process commonly referred to as "antigen retrieval." Because mass spectrometry depends on the ionizability of a substance, and in the case of proteins, the ionizability of the proteins, it is possible to perform mass spectrometry on proteins since their primary structure is maintained and it is this primary structure that is analyzed using mass spectrometry (and associated methods). Because the methods such as mass spectrometry use known sequences of protein fragments to identify the proteins (e.g., peptide fingerprinting), mass spectrometry now can be successfully applied to articles derived from paraffin-embedded tissue samples.

Thus, the antigen retrieval step (decrosslinking) of the present invention unlocks a wealth of untapped proteomic information by enabling the analysis of previously chemically fixed paraffin-embedded tissue samples, including tissue microarrays (TMAs). In preferred embodiments, the use of mass spectrometric analysis techniques allow the simultaneous identification of multiple proteins.

Reversing the chemical crosslinks (i.e., breaking the bonds formed from chemically crosslinking the analyte or "decrosslinking") can occur through a variety of techniques. For example, it can occur through the application of energy in the presence of water or buffer at a range of pH values. The energy applied can be heat or radiation. Preferably, the conditions are selected in the reversing step such that substantially no naturally occurring bonds in the analyte are broken.

In certain embodiments, the method can further include cleaving at least a portion of the naturally occurring bonds (or other bonds not formed by the chemical fixative) in the decrosslinked analyte to form analyte fragments. For proteins, typically the cleavage occurs with an enzyme, such as trypsin, or by chemical cleaving reagents, such as cyanogen bromide. This cleavage step can occur prior to or after decrossinking, although it is preferred that this step be carried out after decrosslinking. Chemical and/or enzymatic cleavage results in fragments of the analyte, e.g., peptides from proteins that are amenable to analysis by methods dependent on their primary structure, such as mass spectrometry. Furthermore, although not preferred, the decrosslinking step could also result in fragmentation of the analytes in addition to decrosslinking. In certain embodiments, analyzing the decrosslinked analyte can include identifying and/or quantifying the decrosslinked analyte. According to the invention, the method comprises analyzing mass spectra. A digital computer forms a classification model that can be used to differentiate classes of samples associated with different biological statuses. The classification model can be used as a diagnostic tool for prediction. It may also be used to identify potential markers associated with a biological status. In addition, the classification model can be formed using a process such as, for example, a recursive partitioning process.

According to the invention, the method comprises: entering into a digital computer a data set obtained from mass spectra from a plurality of the cellular samples, wherein each sample is, or is to be assigned to a class within a class set comprising two or more classes, each class characterized by a different biological status, and wherein each mass spectrum comprises data representing signal strength as a function of mass-to-charge ratio or a value derived from mass-to-charge ratio; and b) forming a classification model which discriminates between the classes in the class set, wherein forming comprises analyzing the data set by executing code that embodies a classification process comprising a recursive partitioning process.

Another example of the invention is directed to a method for classifying an unknown sample into a class characterized by a biological status using a digital computer. The method comprises: a) entering data obtained from a mass spectrum of the unknown sample into a digital computer; and b) processing the mass spectrum data using a classification model to classify the unknown sample in a class characterized by a biological status. The classification model may be formed using a recursive partitioning process.

A variety of different types of cellular samples (e.g., tissue and/or individual cells) can be used, including microarrays. In the preferred embodiment where the specimen under analysis is a microarray, at least one sample is from a human. In another aspect, at least one sample is from a plant. In another aspect, at least one sample is from an insect. In another aspect, at least one sample is from an individual having a disease. In a further aspect, the disease is a progressive disease and the sample is a microarray that includes a plurality of samples representing different stages in the progression of the disease. In one aspect, the disease is cancer. In another aspect, the disease is a respiratory disease, an infectious disease, an immune disease, a disease affecting reproductive organs (male or female), a cardiovascular disease, a disease affecting the endocrine system, a disease affecting the urinary system, a disease affecting the digestive system, a neurodegenerative disease and/or a neuropsychiatric disease. In the case of a chronic disease, the microarray can include samples representing both remission periods and exacerbation periods.

Similar variation in types and disease status can be applied to samples from a variety of experimental animals, e.g., mouse or rabbit. Individual tissues or collections of tissues, as in tissue microarrays, can be analyzed in a manner identical to human tissue, reflecting the utility of the method in drug target identification and/or validation. Preferably, the non-human animal is an animal model for a disease. In another aspect, the non-human animal includes at least one cell having therein exogenous nucleic acid (i.e., a nucleic acid which is not naturally found in the genome of an animal or plant).

In a further aspect, the non-human animal has been treated with a therapy for treating the disease.

The following definitions are provided for specific terms that are used in the following written description.

The terms "comprises" and variations thereof do not have a limiting meaning where these terms appear in the description and claims.

As used herein, "a," "an," "the," "at least one," and "one or more" are used interchangeably. Thus, for example, a sample that comprises a chemically crosslinked analyte can be interpreted to mean that the sample includes "one or more" such analytes.

As used herein, "analyte" shall mean a molecule, compound, composition, or complex, either naturally occurring or synthesized, to be detected or measured in or separated from a sample of interest. Analytes include, without limitation, proteins, peptides, amino acids, fatty acids, nucleic acids, carbohydrates, hormones, steroids, lipids, vitamins, bacteria, viruses, pharmaceuticals, and metabolites. These analytes may or may not be capable of being crosslinked by a chemical fixative. For example, certain analytes, such as pharmaceuticals, metabolites, and vitamins, may not be chemically crosslinked, but can be analyzed in the method.

As used herein, "chemically crosslinked analyte" is an analyte that has been crosslinked using chemical means as a result of the addition of a chemical fixative capable of crosslinking, such as formalin or glutaraldehyde, for example. This does not include ethanol fixation. That is, although the analyte may have crosslinks within the molecule prior to addition of a chemical fixative, additional "chemical crosslinks" are incorporated into the analyte using a chemical crosslinking reagent (e.g., fixative).

As used herein, "a cellular sample" is one that is biological in nature in that it includes cells, whether they are individual cells, a part of a tissue, or a part of an organ. It is a recognized practice to isolate cells, e.g., from a biological fluid, form aggregates of the cells, e.g., by centrifugation, and to create chemically fixed paraffin embedded sections of the cell aggregates, commonly referred to as "cell blocks". The cells within the cell block reflect their tissues and organs of origin.

As used herein, a "tissue" is an aggregate of cells that perform a particular function in an organism and generally refers to cells and cellular material (e.g., such as extracellular matrix material) from a particular physiological region. The cells in a particular tissue can include several different cell types. A non-limiting example of this would be brain tissue that further includes neurons and glial cells, as well as capillary endothelial cells and blood cells.

As used herein, "chemically fixed, paraffin-embedded tissue section" refers to a chemically fixed, paraffin-embedded, material, such as formalin-fixed paraffin-embedded tissue. This term is often used conventionally to refer to tissues, cells, or organs embedded in paraffin. Herein, this is also referred to as "chemically fixed, paraffin-embedded cellular sample." While referred to as a "section," the embedded tissue or cell(s) can be generally of any shape or size, and are generally 20 microns or less in thickness.

As used herein, "a tissue microarray" is a microarray that includes a plurality of microscopic locations, each location comprising tissue cells and/or extracellular materials from tissues, or cells typically infiltrating tissues, where the morphological features of the cells or extracellular materials at each location are visible through microscopic examination. The term "microarray" implies no upper limit on the size of the tissue sample on the microarray, but merely encompasses a plurality of cellular (e.g., tissue) samples that, in one aspect, can be viewed using a microscope. As used herein "different types of tissues" refers to tissues which are preferably from different organs or which are at least from anatomically and histologically distinct sites in the same organ.

The above summary of the present invention is not intended to describe each disclosed embodiment or every implementation of the present invention. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list.

The invention is directed to the analysis of cellular samples (e.g., cells, tissues, organs) that include a chemically crosslinked analyte (e.g., formalin-fixed proteins), wherein the sample is embedded in an organic solid material (e.g., paraffin) involving the use of mass spectrometry. The method can be used on tissue microarrays comprised of formalin-fixed paraffin embedded tissue with or without enzymatic degradation to distinguish various tumor types from one another. This represents a potentially useful approach to the growing field of "theranostics" - diagnosis of proper therapy for a given disease.

The cellular sample is one that is biological in nature in that it includes cells, whether they are individual cells, a part of a tissue, or a part of an organ. The cellular sample preferably includes a tissue section. Preferably, the cellular sample includes formalin-fixed tissue.

In a particularly preferred embodiment, the invention provides a method of analyzing a chemically fixed, paraffin-embedded, tissue section using mass spectrometry. The tissue samples analyzed by the method of the present invention can be evaluated in high throughput parallel analyses using MALDI mass spectrometry, enabling gene identification, protein identification, molecular profiling, selection of promising drug targets, sorting and prioritizing of expressed sequence array data, and the identification of abnormal physiological processes associated with disease.

In a preferred embodiment, a cellular sample (e.g., tissue sample) that has been chemically crosslinked (e.g., fixed with formalin), embedded in an organic solid material (e.g., paraffins), formed either into a block or a microarray, both of which are then typically formed into 5-micron thick sections, can be subjected to a process that makes available an analyte by reversing at least a portion of the chemical crosslinks to form decrosslinked analyte, such as the process described in Applicants' copending patent application, U.S. Patent Publication No. 2005/0130121-A1, entitled Analysis of Chemically Crosslinked Cellular Samples.

The process is preferably accomplished while substantially no naturally occurring bonds (or other bonds present prior to crosslinking) in the analyte are cleaved. If desired, the sample can be separated from the solid organic material (e.g., paraffin). This can occur prior to reversing the crosslinking. It can be accomplished by steam or any heating method. Preferably, this occurs at a temperature below that which causes decrosslinking.

A variety of techniques can be used to reverse at least a portion of the chemical crosslinks. Preferably, this is done through the application of energy. This can be accomplished in the presence of water or buffer at a range of pH values. The energy can be heat or radiant energy. Other methods can also be used including the use of chemical reagents, including acids such as citric acid. Such techniques are described in Shi S-R, Cote RJ, Taylor CR., "Antigen retrieval immunohistochemistry: past, present, and future," J Histochem Cytochem 1997; 45(3):327-343.

This decrosslinked analyte can be directly subjected to analysis by a method such as mass spectrometry. Alternatively, the decrosslinked analyte can be subjected to a process for cleaving at least a portion of the naturally occurring bonds (or other bonds present prior to crosslinking) within the analyte. This can be done chemically or enzymatically (e.g., using trypsin), for example.

Optionally, the decrosslinked and/or cleaved analyte can be treated or tagged with a molecular probe (e.g., a dye) that can assist in enhancing or suppressing signal intensity of the analyte in a controlled manner. Such reagents and methods are well-known to one of skill in the art. For example, tagging the phosphopeptides can occur through various well-known methods such as Immobilized Metal Affinity Chromatography (IMAC). As used herein a "molecular probe" is any detectable molecule or molecule which produces a detectable signal upon reacting with a biological molecule. "Reacting" encompasses binding, labeling, or initiating an enzymatic reaction. Such detectable molecular probe can be recognized by a detectable binding reagent. In this context, a "detectable binding reagent" refers to an agent that specifically recognizes and interacts or binds with a molecular probe associated with an analyte one wishes to measure, wherein the agent has a property permitting detection when bound. "Specifically recognize and interact" means that a binding agent interacts with the molecular probe associated with the analyte one wishes to measure, to the substantial exclusion of other analytes also present in the sample. A detectable binding reagent can possess an intrinsic property that permits direct detection, or it can be labeled with a detectable moiety. As used herein, "detectable moiety" refers to a moiety that can be attached to a binding reagent that confers detection of the binding reagent by a particular method or methods. Detectable moieties include, but are not limited to, radiolabels (e.g., ³²P, ³⁵S, ¹²⁵I, etc.), enzymes (e.g., alkaline phosphatase, peroxidase, etc.), fluorophores (e.g., fluorescein, amino coumarin acetic acid, tetramethylrhodamine isothiocyanate (TRITC), Texas Red, Cy3.0, Cy5.0, green fluorescent protein, etc.) and colloidal metal particles.

The method preferably includes cleaving at least a portion of other bonds (e.g., naturally occurring bonds or other bonds within the analyte prior to crosslinking) in the decrosslinked analyte to form analyte fragments. These analyte fragments can then be analyzed. Cleaving at least a portion of the bonds in the decrosslinked analyte includes contacting the decrosslinked analyte with an enzyme or chemical reagent. Preferably, an enzyme is used, such as trypsin, pepsin, pronase, chymotrypsin, and combinations thereof.

This cleavage step can occur prior to or after decrossinking, athough it is preferred that this step be carried out after decrosslinking. Chemical and/or enzymatic cleavage results in fragments of the analyte, e.g., peptides from proteins that are amenable to analysis by methods dependent on their primary structure, such as mass spectrometry. Furthermore, although not preferred, the decrosslinking step could also result in fragmentation of the analytes in addition to decrosslinking.

It should be understood that cellular samples can include analytes that are not crosslinked and subsequently decrosslinked. For example, certain analytes, such as pharmaceuticals, metabolites, and vitamins, may not be chemically crosslinked. Such analytes can also be analyzed along with the decrosslinked analytes using the methods of the present invention.

The digest can be directly subjected to mass spectrometry, or an eluate of the digest can be removed and this can be subjected to a method of analysis. In a preferred embodiment, the method of analysis is mass spectrometry.

In embodiments of the invention, a data set obtained from mass spectra is entered into a digital computer to form a classification model by the method described in U.S. Patent No. 6,675,104. The mass spectra are preferably obtained from tissue samples having known characteristics. In preferred embodiments, the data set used to form the classification model is characterized as a "known" data set, because the biological statuses associated with the biological samples are known before the data set is used to form the classification model. In comparison, an "unknown" data set includes data that is obtained from mass spectra of samples where it is unclear if the samples are associated with the biological statuses which are discriminated by the classification model when the mass spectra are formed. Unknown data may be derived from a biological sample from a test patient who is to be diagnosed using the classification model. In some environments, the known data set is referred to as "training data". In some embodiments of the invention, the data set used to form the classification model may be an unknown data set.

In embodiments of the invention, each cellular sample used is, or is to be assigned to a class of a set of two or more classes, and each class is characterized by a different biological status. For example, a first class of samples may be associated with a biological status such as a diseased state. A second class of mass spectra of samples may be associated with a biological status such as a non-diseased state. The samples in the first and second classes may form the class set. The mass spectra from each of the respective classes can contain data that differentiates the first and the second classes.

In embodiments of the invention, each mass spectrum in the analyzed mass spectra could comprise signal strength data as a function of time-of-flight, a value derived from time-of-flight (e.g. mass-to-charge ratio, molecular weight, etc.), mass-to-charge ratio, or a value derived from mass-to-charge ratio (e.g., molecular weight). As known by those of ordinary skill in the art, mass-to-charge ratio values obtained from a time-of-flight mass spectrometer are derived from time-of-flight values. Mass-to-charge ratios may be obtained in other ways. For example, instead of using a time-of-flight mass spectrometer to determine mass-to-charge ratios, mass spectrometers using quadrupole analyzers and magnetic mass analyzers can be used to determine mass-to-charge ratios.

In preferred embodiments, each mass spectrum comprises signal strength data as a function of mass-to-charge ratio. In a typical spectral view-type mass spectrum, the signal strength data may be in the form of "peaks" on a graph of signal intensity as a function of mass-to-charge ratio. Each peak may have a base and an apex, where peak width narrows from the base to the apex. The mass-to-charge ratio generally associated with the peak corresponds to the apex of the peak. The intensity of the peak is also generally associated with the apex of the peak.

Generally, the mass-to-charge ratio relates to the molecular weight of a potential marker. For example, if a potential marker has a charge of +1, then the mass-to-charge ratio is equal to the molecular weight of the potential marker represented by the signal. Thus, while some mass spectra plots may show signal intensity as a function of molecular weight, the molecular weight parameter is in fact derived from mass-to-charge ratios.

While many specific embodiments of the invention discussed herein refer to the use of mass-to-charge ratios, it is understood that time-of-flight values, or other values derived from time-of-flight values, may be used in place of mass-to-charge ratio values in any of the specifically discussed exemplary embodiments.

The data set may comprise any suitable data and may be entered automatically or manually into a digital computer. The data may be raw or preprocessed before being processed by the classification process run on the digital computer. For example, the raw intensities of signals at predetermined mass-to-charge ratios in the mass spectra may be used as the data set. Alternatively, the raw data may be preprocessed before the classification model is formed. For example, in some embodiments, the log values of the intensities (e.g., base 2) of the signals in the mass spectra may be used to form the data set.

The data set is entered into the digital computer. Computer code that embodies a classification process uses the data set to form a classification model. Exemplary classification processes include hierarchical classification processes such as a classification and regression tree process, multivariate statistical analyses such as a cluster analysis, and non-linear processes such as a neural network analysis. In preferred embodiments, the data set is processed using a classification and regression tree process to produce a classification model such as a classification and regression tree. These and other classification processes and classification models are described in greater detail below.

The created classification model may be predictive or descriptive. For example, the model can be used to predict whether an unknown test biological sample is or is not associated with a particular biological status. Alternatively or additionally, the classification model may be interrogated to identify features in the data that differentiate the biological status(s) being analyzed. A feature includes any aspect of the mass spectra data that can differentiate the particular classes being analyzed. Suitable features that can be identified include, but are not limited to, signal intensities or signal intensity ranges at one or more mass-to-charge ratios, signal shapes (e.g., peak shapes), signal areas (e.g., peak areas), signal widths (e.g., peak widths such as at the bottom of a peak), the number of signals in each mass spectrum, etc. In a typical example, the classification model may indicate that a feature such as a particular signal intensity at a given mass-to-charge ratio differentiates diseased samples from non-diseased samples. In yet another example, the classification model may indicate that a combination of features differentiates diseased samples from non-diseased samples. For example, signal intensity ranges for two or more signals at different mass-to-charge ratios may differentiate a diseased state from a non-diseased state.

As used herein, "biological status" of a sample refers to any characterizing feature of a biological state of the sample or the organism or source from which the sample is derived. The feature can be a biological trait such as a genotypic trait or a phenotypic trait. The feature can be a physiological or disease trait, such as the presence or absence of a particular disease, including infectious disease. The feature also can be a condition (environmental, social, psychological, time-dependent, etc.) to which the sample has been exposed.

Genotypic traits can include the presence or absence of a particular gene or polymorphic form of a gene; or combination of genes. Genetic traits may be manifested as phenotypic traits or exist as susceptibilities to their manifestation, such as a susceptibility to a particular disease (e.g., a propensity for certain types of cancer or heart disease).

Phenotypic traits include, for example, appearance, physiological traits, physical traits, neurological conditions, psychiatric conditions, response traits, e.g., or response or lack of response to a particular drug. Phenotypic traits can include the presence of absence of so-called "normal" or "pathological" traits, including disease traits. Another status is the presence or absence of a particular disease. A status also can be the status of belonging to a particular person or group such as different individuals, different families, different age states, different species, and different tissue types.

In some embodiments, the biological statuses may be, for example, one or more of the following in any suitable combination: a diseased state, a normal status, a pathological status, a drug state, a non-drug state, a drug responder state, a non-drug responder state, and a benign state. A drug state may include a state where patient who has taken a drug, while a non-drug state may include a state where a patient has not taken a drug. A drug responder state is a state of a biological sample in response to the use of a drug. Specific examples of disease states include, e.g., cancer, heart disease, autoimmune disease, viral infection, Alzheimer's disease and diabetes. More specific cancer statuses include, e.g., prostate cancer, bladder cancer, breast cancer, colon cancer, and ovary cancer. Biological statuses may also include beginning states, intermediate states, and terminal states. For example, different biological statuses may include the beginning state, the intermediate state, and the terminal state of a disease such as cancer.

Other statuses may be associated with different environments to which different classes of samples are subjected. Illustrative environments include one or more conditions such as treatment by exposure to heat, electromagnetic radiation, exercise, diet, geographic location, etc. For example, a class of biological samples (e.g., all blood samples) may be from a group of patients who have been exposed to radiation and another class of biological samples may be from a group of patients who have not been exposed to radiation. The radiation source may be an intended radiation source such as an x-ray machine or may be an unintended radiation source such as a cellular phone. In another example, one group of persons may have been on a particular diet of food, while another group may have been on a different diet.

In other embodiments of the invention, the different biological statuses may correspond to samples that are associated with respectively different drugs or drug types. In an illustrative example, mass spectra of samples from persons who were treated with a drug of known effect are created. The mass spectra associated with the drug of known effect may represent drugs of the same type as the drug of known effect. For instance, the mass spectra associated with drugs of known effect may represent drugs with the same or similar characteristics, structure, or the same basic effect as the drug of known effect. Many different analgesic compounds, for example, may all provide pain relief to a person. The drug of known effect and drugs of the same or similar type might all regulate the same biochemical pathway in a person to produce the same effect on a person. Characteristics of the biological pathway (e.g., up- or down-regulated proteins) may be reflected in the mass spectra.

A classification model can be created using the mass spectra associated with the drug of known effect and mass spectra associated with different drugs, different drug types, or no drug at all. Once the classification model is created, a mass spectrum can then be created for a candidate sample associated with a candidate drug of unknown effect. Using the classification model, the mass spectrum associated with the candidate sample is classified. The classification model can determine if the candidate sample is associated with the drug of known effect or another drug of a different type. If, for example, the classification model classifies the candidate sample as being associated with the drug of known effect, then the candidate drug is likely to have the same effect on a person as the drug of known effect. Accordingly, embodiments of the invention can be used, among other things, to discover and/or characterize drugs.

In embodiments of the invention, a gas phase ion spectrometer mass may be used to create mass spectra. A "gas phase ion spectrometer" refers to an apparatus that measures a parameter that can be translated into mass-to-charge ratios of ions formed when a sample is ionized into the gas phase. This includes, e.g., mass spectrometers, ion mobility spectrometers, or total ion current measuring devices.

The mass spectrometer may uses matrix-assisted laser desorption/ionization (MALDI).

In some embodiments, an ion mobility spectrometer can be used to detect and characterize a marker. The principle of ion mobility spectrometry is based on the different mobility of ions. Specifically, ions of a sample produced by ionization move at different rates due to their difference in, e.g., mass, charge, or shape, through a tube under the influence of an electric field. The ions (typically in the form of a current) are registered at a detector and the output of the detector can then be used to identify a marker or other substances in the sample. One advantage of ion mobility spectrometry is that it can be performed at atmospheric pressure.

A laser desorption time-of-flight mass spectrometer is used to create the mass spectra. Laser desorption spectrometry is especially suitable for analyzing high molecular weight substances such as proteins. For example, the practical mass range for a MALDI can be up to 300,000 daltons or more. Moreover, laser desorption processes can be used to analyze complex mixtures and have high sensitivity. In addition, the likelihood of protein fragmentation is lower in a laser desorption process such as a MALDI than in many other mass spectrometry processes. Thus, laser desorption processes can be used to accurately characterize and quantify high molecular weight substances such as proteins.

In a typical process for creating a mass spectrum, a probe with a marker is introduced into an inlet system of the mass spectrometer. The marker is then ionized. After the marker ions are generated, the generated ions are collected by an ion optic assembly, and then a mass analyzer disperses and analyzes the passing ions. The ions exiting the mass analyzer are detected by a detector. In a time-of-flight mass analyzer, ions are accelerated through a short high voltage field and drift into a high vacuum chamber. At the far end of the high vacuum chamber, the accelerated ions strike a sensitive detector surface at different times. Since the time-of-flight of the ions is a function of the mass-to-charge ratio of the ions, the elapsed time between ionization and impact can be used to identify the presence or absence of molecules of specific mass-to-charge ratio. The time of flight data may then be converted into mass-to-charge ratios to generate a spectrum showing the signal strength of the markers as a function of mass-to-charge ratio.

Mass spectra data generated by the desorption and detection of markers can be preprocessed using a digital computer after or before generating a mass spectra plot. Data analysis can include the steps of determining the signal strength (e.g., height of signals) of a detected marker and removing "outliers" (data deviating from a predetermined statistical distribution). For example, the observed signals can be normalized. Normalization is a process whereby the height of each signal relative to some reference is calculated. For example, a reference can be background noise generated by instrument and chemicals (e.g., an energy absorbing molecule) which is set as zero in the scale. Then, the signal strength detected for each marker or other substances can be displayed in the form of relative intensities in the scale desired (e.g., 100). Alternatively, a standard may be admitted with the sample so that a signal from the standard can be used as a reference to calculate relative intensities of the signals observed for each marker or other markers detected.

### Forming the Data Set

Once the mass spectra are obtained, a data set such as a known data set is formed. The data set comprises data that is obtained from the mass spectra of the class set of biological samples. The mass spectra data forming the data set can be raw, unprocessed data. For example, raw signal intensity values at identified mass values from the mass spectra may be used to form the data set. In another example, raw signal patterns from mass spectra may be used to form the data set.

In alternative embodiments, data may be preprocessed before it is used to form the classification model. The mass spectra may then be processed in any suitable manner before being used to form the classification model. For example, the signals in the mass spectra may be processed by taking the log values of the signal intensities, removing outliers, removing signals which are less likely to be associated with potential markers, removing signals which have low intensities, etc.

In some embodiments, the data set may comprise raw or preprocessed pattern data that relates to the particular pattern of each mass spectrum. For example, for a mass spectrum comprising many signal peaks, the pattern of the signal peaks may constitute a fingerprint for the biological sample used to create the mass spectrum. The classification process can classify the different spectra according to patterns or pattern segments that may be common to the spectra in the respectively different classes differentiated by the classification model. A computer program such as a neural network program, for example, can receive plural mass spectra of known samples associated with known biological statuses. The neural network can be trained with the mass spectra data so that it can differentiate between mass spectra patterns belonging to the respectively different classes. The trained neural network can then be used to classify a mass spectrum associated with an unknown sample based on the pattern in the mass spectrum.

In other embodiments, the data set comprises data relating to the intensities of the signals in the mass spectra. In these embodiments, some or all of the signals in each mass spectrum may be used to form the data set. For example, the intensities of less than all of the signals (e.g., peaks) in a spectra view type mass spectrum can be used to form the data set. In preferred embodiments, mass-to-charge ratios are identified, and the identified mass-to-charge ratios are used to select signals from the mass spectra. The intensities of these selected signals can be used to form the data set. By using data from less than all signals in each mass spectrum to form the data set, the number of data points that will be processed is reduced so that data processing occurs more rapidly. Data of signals that have a low likelihood of representing acceptable markers may be excluded from the data set.

Mass-to-charge ratios may be identified in any number of ways. For example, the mass-to-charge ratios may be identified by comparing the mass spectra of different classes having different biological statuses. The mass-to-charge ratios of signals that are likely to differentiate the classes may be selected. The comparison may be performed manually (e.g., by a visual comparison) or may be done automatically with a digital computer. For example, mass spectra associated with different classes of samples can be visually compared with each other to determine if the intensity of a signal at a mass-to-charge ratio in a mass spectrum from one sample class is significantly greater than or less than a signal at the same mass-to-charge ratio in a mass spectrum from a different sample class, thus indicating potential differential expression. Mass-to-charge ratios where these signal differences occur may be selected.

Alternatively or additionally, certain predefined criteria may be provided to first select certain signals or signal clusters. The selected signal clusters may then be used to identify particular mass-to-charge ratios. For example, signals or signal clusters having a signal intensity or average signal intensity above or below a certain signal intensity threshold may be automatically selected. Mass-to-charge ratios associated with these selected signals or signal clusters may then be identified.

Once mass-to-charge ratios are identified, intensity values are determined for each signal at the identified mass values for all mass spectra. The intensity value for each of the signals is normalized from 0 to 100 to remove the effects of absolute magnitude. Then, the logarithm (e.g., base 2) is taken for each normalized signal intensity. Taking the logarithm of the signal intensities removes skew from the measurements.

The log normalized data set is then processed by a classification process that can be embodied by code that is executed by a digital computer. After the code is executed by the digital computer, the classification model is formed.

### Forming the Classification Model

A classification process embodied by code that is executed by a digital computer can process the data set. The code can be executed by the digital computer to create a classification model. The code may be stored on any suitable computer readable media. Examples of computer readable media include magnetic, electronic, or optical disks, tapes, sticks, chips, etc. The code may also be written in any suitable computer programming language including, C, C++, etc.

The digital computer may be a micro, mini or large frame computer using any standard or specialized operating system such as a WINDOWS based operating system. In other embodiments, the digital computer may simply be one or more microprocessors. The digital computer may be physically separate from the mass spectrometer used to create the mass spectra. Alternatively, the digital computer may be coupled to or physically incorporated into the mass spectrometer. Mass spectra data can be transmitted from the mass spectrometer to the digital computer manually or automatically. For example, in one embodiment, a known data set may first be obtained from a plurality of mass spectra. The known data set may then be manually entered into a digital computer running code that embodies a classification process. In another embodiment, the generation and/or collection of mass spectra data, the preprocessing of the data, and the processing of the preprocessed data by a classification process may be performed using the same physical computational apparatus.

In embodiments of the invention, additional data may be used to from the classification model. The additional data may or may not relate to mass spectra. For instance, in some embodiments, pre-existing marker data may be used in addition to a known data set to form the classification model.

Any suitable classification process may be used in embodiments of the invention. For example, the classification process may be a hierarchical classification process such as a classification and regression tree process or a multivariate statistical analysis. A multivariate statistical analysis looks at patterns of relationships between several variables simultaneously. Examples of multivariate statistical analyses include well known processes such as discriminate function analysis and cluster analysis.

In embodiments of the invention, the classification process preferably includes a hierarchical, recursive partitioning process such as a classification and regression tree process. In embodiments of the invention, the classification and regression tree process is embodied by computer code that can be executed by a digital computer. Examples of exemplary classification and regression tree processes appropriate for use with the present invention include those described in U.S. Patent No. 6,675,104.

The classification model may be used to classify an unknown sample into a biological status. In this method the mass spectrum of a test sample can be compared to the classification model associated with a particular biological status to determine whether the sample can be properly classified with the biological status. A mass spectrum of the unknown biological sample can be obtained, and data obtained from a mass spectrum of the unknown sample can be entered into a digital computer. The entered data may be processed using a classification model. The classification model may then classify the unknown sample into a particular class. The class may have a particular biological status associated with it, and the person can be diagnosed as having that particular biological status.

This method has particular use for clinical applications. For example, in the process of drug discovery, one may wish to determine whether a candidate molecule produces the same physiological result as a particular drug or class of drugs (e.g., the class of seratonin re-uptake inhibitors) in a biological system. A classification model is first developed that discriminates biological systems based on exposure to the drug or class of drugs of interest (e.g., persons or test animals). Then, the biological system is exposed to the test molecule and a mass spectrum of a sample from the system is produced. This spectrum is then classified as belonging or not belonging to the classification of known drug or group of drugs against which it is being tested. If the candidate molecule is assigned to the class, this information is useful in determining whether to perform further research on the drug.

Other potential applications include a classification model that discriminates various toxic and non-toxic biological states; a classification model that discriminates between persons who are responders and non-responders to a particular drug; and/or a classification model that distinguishes person having a disease from those who do not have the disease. Thus, this method is useful for clinical diagnostics.

One embodiment is directed to analyzing cancer. Pathologists grade cancers according to their histologic appearance. Features of low-grade cancers include enlarged nuclei with a moderate increase in nuclear/cytoplasmic ratio, small number of mitoses, moderate cytologic heterogeneity, and retention of generally normal architecture. Features of high-grade cancers include enlarged, bizarre looking nuclei with a high nuclear/cytoplasmic ratio; increased number of mitoses, some of which may appear atypical; and little or no resemblance to normal architecture. It is useful to develop a classification model that distinguishes a biological sample coming from un-diseased, low-grade cancer, and high-grade cancer, since this diagnosis often dictates therapeutic decisions as well as can predict prognosis. The sample can be a solid tissue biopsy or a fine needle aspirate of the suspected lesion. However, in another embodiment, the samples can derive from more easily collected sources from the group of individuals being tested, such as urine, blood or another body fluid. This is particularly useful for cancers that secrete cells or proteins into these fluids, such as bladder cancer, prostate cancer and breast cancer. Upon establishment of the classification model for these states, the model can be used to classify a sample from a person subject to diagnostic testing. In another application, a classification model is developed that discriminates between classes of individuals having a particular physical or physiological trait that is not pathologic. Then, individuals unknown to have the trait can be classified by testing a sample from the individual and classifying a spectrum into the class having the trait, or outside the class having the trait.

The classification model can also be used to estimate the likelihood that an unknown sample is accurately classified as belonging to a class characterized by a biological status. For instance, in a classification and regression tree, the likelihood of potential misclassification can be determined. Illustratively, a classification and regression tree model that differentiates a diseased state from a non-diseased state classifies an unknown sample from a patient.

### Systems Including Computer Readable Media

The invention method may be performed on systems including a computer readable medium. The computer readable medium may be used for storing instructions to be executed by the digital computer.

The mass spectrometer can be operably associated with a digital computer without being physically or electrically coupled to the digital computer. For example, data from the mass spectrometer could be obtained (as described above) and then the data may be manually or automatically entered into the digital computer using a human operator. In other embodiments, the mass spectrometer can automatically send data to the digital computer where it can be processed. For example, the mass spectrometer can produce raw data (e.g., time-of-flight data) from one or more biological samples. The data may then be sent to the digital computer where it may be pre-processed or processed. Instructions for processing the data may be obtained from the computer readable medium. After the data from the mass spectrometer is processed, an output may be produced and displayed on a display.

A computer readable medium may contain any suitable instructions for processing the data from the mass spectrometer. For example, the computer readable medium may include computer code for entering data obtained from a mass spectrum of an unknown biological sample into the digital computer. The data may then be processed using a classification model. The classification model may estimate the likelihood that the unknown sample is accurately classified into a class characterized by a biological status.

### Simple Preparation

In one aspect, the samples are tissue samples. Tissue samples can be obtained from chemically fixed, paraffin-embedded, tissue, and in particular, formalin-fixed, paraffin-embedded, tissue. A chemically-fixed, paraffin-embedded, tissue sample according to the invention typically includes one or more sections derived from tissue and/or cells. Preferably, each sample has at least one known biological characteristic (e.g., such as tissue type or cell type or patient source).

The tissue can be in the form of a tissue microarray, such as those described in Kononen et al., 1998, Nat. Med. 4: 844-7. Generation of microarrays can be partially or fully automated using tissue microarrayers, such as the ones described in WO 99/44062, WO 99/44063, and U.S. Pat. No. 6,136,592.

Cells also can be obtained to provide one or more samples. Cells typically are formed into paraffin sections by centrifugation. Cells can be obtained from suspensions of cells from tissues (e.g., from a suspension of minced tissue cells, such as from a dissected tissue), from bodily fluids (e.g., blood, plasma, sera, and the like), from mucosal scrapings (e.g., such as from buccal scrapings or pap smears), and/or from other procedures such as bronchial ravages, amniocentesis procedures, and/or leukophoresis. In some aspects, cells are cultured first prior to being made part of the sample to expand a population of cells to be analyzed. Cells from continuously growing cell lines, from primary cell lines, and/or stem cells, also can be used.

In one aspect, a sample includes a plurality of tissues/cells from a single individual, i.e., the sample is microarray representing the "whole body" of an individual. Tissues can be selected from the group consisting of skin, neural tissue, cardiac tissue, liver tissue, stomach tissue, large intestine tissue, colon tissue, small intestine tissue, esophagus tissue, lung tissue, cardiac tissue, spleen tissue, pancreas tissue, kidney tissue, tissue from a reproductive organ(s) (male or female), adrenal tissue, and the like. Tissues from different anatomic or histological locations of a single organ can also be obtained, e.g., such as from the cerebellum, cerebrum, and medulla, where the organ is the brain. Some microarrays include samples representative of organ systems (i.e., comprising samples from multiple organs within an organ system), e.g., the respiratory system, urinary system, kidney system, cardiovascular system, digestive system, and reproductive system (male or female). In a preferred aspect, a whole body microarray additionally comprises a sample of cells from a bodily fluid of the patient (e.g., from a blood sample).

The microarray also can include a plurality of cells from individuals sharing a trait. For example, the trait shared can be gender, age, pathology, predisposition to a pathology, exposure to an infectious disease (e.g., HIV), kinship, death from the same disease, treatment with the same drug, exposure to chemotherapy, exposure to radiotherapy, exposure to hormone therapy, exposure to surgery, exposure to the same environmental condition (e.g., such as carcinogens, pollutants, asbestos, TCE, perchlorate, benzene, chloroform, nicotine and the like), the same genetic alteration or group of alterations, expression of the same gene or sets of genes (e.g., samples can be from individuals sharing a common haplotype, such as a particular set of HLA alleles), and the like.

Samples can be obtained from an individual with a disease or pathological condition, including, but not limited to: a blood disorder, blood lipid disease, autoimmune disease, bone or joint disorder, a cardiovascular disorder, respiratory disease, endocrine disorder, immune disorder, infectious disease, muscle wasting and whole body wasting disorder, neurological disorders including neurodegenerative and/or neuropsychiatric diseases, skin disorder, kidney disease, scleroderma, stroke, hereditary hemorrhage telangiectasia, diabetes, disorders associated with diabetes (e.g., PVD), hypertension, Gaucher's disease, cystic fibrosis, sickle cell anemia, liver disease, pancreatic disease, eye, ear, nose and/or throat disease, diseases affecting the reproductive organs, gastrointestinal diseases (including diseases of the colon, diseases of the spleen, appendix, gall bladder, and others), and the like. For further discussion of human acme diseases, see Mendelian Inheritance in Man: A Catalog of Human Genes and Genetic Disorders by Victor A. McKusick (12th Edition (3 volume set) June 1998, Johns Hopkins University Press, ISBN: 0801857422). Preferably, samples from a normal demographically matched individual and/or from a non-disease tissue from a patient having the disease are arrayed on the same or a different microarray to provide controls.

In a preferred aspect, a sample is provided in a microarray format that includes a plurality of cells, which represent different stages of a cell proliferative disorder, such as cancer. In this context, "a cell proliferative disorder" is a condition marked by any abnormal or aberrant increase in the number of cells of a given type or in a given tissue. Cancer is often thought of as the prototypical cell proliferative disorder, yet disorders such as atherosclerosis, restenosis, psoriasis, inflammatory disorders, some autoimmune disorders (e.g., rheumatoid arthritis), are also caused by abnormal proliferation of cells, and are thus examples of cell proliferative disorders.

In one aspect, in addition to including samples, which comprise the primary target of the disease (e.g., such as tumor samples), the microarray includes samples representing metastases of a cancer to secondary tissues/cells. Preferably, the microarray also includes normal tissues from the same patient from whom the abnormally proliferating tissue was obtained. In some aspects, at least one microarray includes cells from a cell line of cancerous cells (either primary or continuous cell lines). Samples can be homogeneous, including a single cell type (e.g., as in a small format or ultrasmall format microarray), or can be heterogeneous, including at least one additional type of cell or cellular material in addition to abnormally proliferating cells (e.g., as in large format microarrays where samples are generally larger than 0.6 mm in diameter). For example, the sample can include abnormally proliferating cells and at least one of fibrous tissue, inflammatory tissue, necrotic cells, apoptotic cells, normal cells, and the like.

Although in a preferred aspect of the invention, the tissue and/or cell samples include human specimens, in one aspect of the invention, specimens from other organisms are used. In one aspect, tissues from non-human animals are used that provide a model of a disease or other pathological condition. When the sample represents specimens from an animal model of a chronic disease, the sample can be in the form of a microarray which includes specimens representing different stages of the disease, e.g., such as from animals in a remission period or an exacerbation period. The microarray can additionally, or alternatively, include tissues from a non-human animal having the disease or condition that has been exposed to a therapy for treating the disease or condition (e.g., drugs, antibodies, protein therapies, gene therapies, antisense therapies, combinations thereof, and the like). In some aspects, the non-human animals can include at least one cell containing an exogenous nucleic acid (e.g., the animals can be transgenic animals, chimeric animals, knockout or knock-in animals). Preferably, arrays from non-human animals include multiple tissues/cell types from such a non-human animal. In one aspect, tissues/cells at different stages of development are used.

In another aspect, samples from plants may be used, such as those discussed in Schumacher U., "Immunohistochemical assessment of cell proliferation in plant tissues using formaldehyde-fixed paraffin-embedded material," Acta Histochem. 1995 Jul:97(3):291-4. Samples may include microarrays that include plants in different stages of their life cycle and/or different types of plant tissues. In some aspects, the plant samples can include at least one cell containing an exogenous nucleic acid (e.g., the plants can be transgenic plants).

In one embodiment, a section of formalin-fixed, paraffin embedded, tissue is obtained and stained with H&E. The stained section is used as a guide to select a region on the tissue section for sampling. While in some aspects, staining with a standard tissue or cell stain such as H&E can be suitable to identify cells or tissue areas of interest, in other aspects, sections of the tissue are evaluated for the expression of one or more biological characteristics (e.g., such as the expression of a genotype, transcript, or peptide, polypeptide, or protein of interest) in the sample represented by the section. An area of interest can be identified which expresses or does not express a particular biological characteristic.

In one embodiment, the sample is prepared by slicing a section of the tissue sample (i.e., cutting transversely from the tissue sample with respect to the longitudinal axis of the sample) and allowed to fall onto a substrate without crumpling. Preferably, each tissue sample generates 150 to 300 sections from 2 to 20 microns thick. More preferably, sections are 4 to 12 microns in thickness.

In some embodiments, an adhesive film is placed on a surface of the tissue sample both to keep the section flat after it is sliced and to provide a surface on which to more easily move the section to a substrate without tearing or wrinkling the section. The section on its adhesive backing is then transferred to a substrate section side-down, and the adhesive film is peeled away from the section. Adhesive films and adhesive-coated slides are both obtainable from Instrumedics, Inc., Hackensack, N.J. (see, e.g., CRYOJAN Tape Transfer System).

It is preferred that the tissue sample be introduced to the substrate in a known pattern for purposes of registration. The initial starting position of the sample, for example, should be known in order to correlate this position with the final position once the substrate size has been reduced to the dimension that will be employed in conducting the assay. Examples include labeling, use of dyes, etc.

Once placed on a substrate, the tissue sample is processed by reversing at least a portion of the chemical crosslinks (i.e., those crosslinks formed by a chemical crosslinking agent such as formalin. This is known conventionally as an antigen retrieval step. Such a process is described in Shi S-R, Cote RJ, Taylor CR., "Antigen retrieval immunohistochemistry: past, present, and future," J Histochem Cytochem 1997; 45(3):327-343. During this decrosslinking step, the chemical fixation is reversed typically through the application of heat in the presence of water. For example, during decrosslinking of formalin-fixed, paraffin embedded, tissue, the tissue sample is subjected to 100°C steam in the presence of citric acid at 9.3 pH. As known by those skilled in the art, modification of the acid used, the temperature and/or the pH will result in varying degrees of reversal of the crosslinking and antigen retrieval. Other energy sources include radiation energy, such as microwave energy.

The tissue section is subject to a process of crosslink reversal (conventionally referred to as antigen retrieval) after affixation to a substrate. In preferred embodiments, the tissue section is affixed to a substrate such as a glass slide before the reversal of crosslinks (decrosslinking).

In a preferred embodiment, the decrosslinked analyte can then be treated with an enzyme or chemical reagent to cleave at least a portion of the naturally occurring bonds or bonds present before crosslinking in the analyte of interest, such as proteins or peptides. Preferably, this involves in situ digestion. Suitable enzymes for cleaving the analyte include, but are not limited to, trypsin, chymotrypsin, pronase, and pepsin. In one embodiment with formalin-fixed, paraffin-embedded, tissue, the enzyme is trypsin. Other agents for cleaving the bonds may also be employed, such as fonnic acid and cyanogen bromide. Such agents and techniques are well-known to one of skill in the art.

### Methods of Use

In one aspect, samples analyzed according to the invention are used to assay the expression and/or form of a cancer-specific marker or tumor-specific antigen. As used herein, "a cancer-specific marker" or a "tumor-specific antigen" is an analyte that is expressed preferentially on cancer cells and tumor cells, respectively, and is not expressed or is expressed to small degree in non-cancer/tumor cells of an adult individual.

In this context, "difference in expression characteristics" or a gene which is "differentially expressed" refers to an increase or decrease in a measurable expression characteristic of a given polypeptide. A difference can be an increase or a decrease in a quantitative measure (e.g., amount of protein or RNA encoding the protein) or a change in a qualitative measure (e.g., location of the protein).

A cancer-specific marker is any analyte that is involved in or correlates with the pathogenesis of a cancer, and can act in a positive or negative manner, as long some aspect of its expression or form influences or correlates with the presence or progression of cancer. While in one aspect, expressed levels of an analyte provide an indication of cancer progression or recurrence, in another aspect of the invention, the expressed form of an analyte provides the indication (e.g., a cleaved or uncleaved state, a phosphorylated or unphosphorylated state).

The cancer-specific marker can be the product of a characterized gene, e.g., such as a cell growth-related polypeptide, which promotes cell proliferation, or can be uncharacterized or only partially characterized (e.g., identified through the use of molecular profiling methods described above). Non-limiting examples of cancer-specific markers include growth factors, growth factor receptors, signal transduction pathway participants, and transcription factors involved in activating genes necessary for cell proliferation.

The so-called tumor antigens are also included among the growth-related polypeptides. Tumor antigens are a class of protein markers that tend to be expressed to a greater extent by transformed tumor cells than by non-transformed cells. As such, tumor antigens can be expressed by non-tumor cells, although usually at lower concentrations or during an earlier developmental stage of a tissue or organism. Tumor antigens include, but are not limited to, prostate specific antigen (PSA; Osterling, 1991, J. Urol. 145: 907-923), epithelial membrane antigen (multiple epithelial carcinomas; Pinkus et al., 1986, Am. J. Clin. Pathol. 85: 269-277), CYFRA 21-1 (lung cancer; Lai et al., 1999, Jpn. J. Clin. Oncol. 29: 421-421) and Ep-CAM (pan-carcinoma; Chaubal et al., 1999, Anticancer Res. 19: 2237-2242). Additional examples of tumor antigens include CA125 (ovarian cancer), intact monoclonal immunoglobulin or light chain fragments (myeloma), and the beta subunit of human chorionic gonadotropin (HCG, germ cell tumors).

In further aspects of the invention, cancer progression can be detected and/or monitored by examining the expression of the activity of a cancer-specific marker. For example, in one aspect, the activity of telomerase is monitored in situ in samples. Methods of in situ detection of telomerase activity are known in the art and are described, for example, in U.S. Pat. No. 6,194,206.

The tissue samples can also be used in conjunction with, or to validate, results obtained through other types of the analyses with the same or other types of samples. For example, the methods of the present invention can be used in conjunction with, or instead of, analyses using in situ detection and visualization using immunohistochemistry; laser capture microdissection (LCM) of samples such as that described in PCT International Application Nos. WO 09917094A2 and WO 098352A1; gel electrophoresis and others, all of which are described in PCT International Application No. WO 02/48674 A2.

Tissue samples prepared according to the present invention also can be used to identify drug targets whose interactions with one or a plurality of analytes are associated with disease. For example, a drug target can be a molecule that is overexpressed or underexpressed during a pathological process. By identifying drug targets, drugs can be screened for which can restore a cell's/tissue's normal physiological functioning. For example, where a drug target is a molecule, which is overexpressed or underexpressed, a suitable drug could be a.molecule (e.g., a therapeutic antibody, polypeptide, or nucleic acid), which restores substantially normal levels of the drug target.

In one aspect, identifying diagnostic analytes is performed by determining which molecules on a microarray are substantially always present in a disease sample and substantially always absent in a healthy sample, or substantially always absent in a disease sample and substantially always present in a healthy sample, or substantially always present in a certain form or amount in a disease sample and substantially always present in a certain other form or amount in a healthy sample. By "substantially always" it is meant that there is a statistically significant correlation between the expression/form of the analyte or set of analytes and the presence of an aberrant physiological process, such as a disease.

Preferably, expression of a diagnostic analytes or set of analytes is examined in a microarray comprising tissues from a drug-treated patient and tissues from an untreated diseased patient and/or from a healthy patient. In this aspect, the efficacy of the drug is monitored by determining whether the expression profile of the diagnostic molecule(s) returns to a profile which is substantially similar (e.g., not significantly different as determined by routine statistical testing) to the expression profile of the same analyte(s) in a healthy patient or a patient who has achieved a desired therapeutic outcome. In one aspect of the invention, data relating to any, or all of, tissue type, stage of development or disease, patient history, family history, diagnosis, prognosis, medication, morphology, concurrent illnesses, expression of molecular characteristics (e.g., markers), and the like, are recorded and stored in a database, indexed according to the tissue sample obtained.

### EXAMPLES

These examples are merely for illustrative purposes only and are not meant to be limiting on the scope of the appended claims. All parts, percentages, ratios, etc. in the examples and the rest of the specification are by weight, unless noted otherwise. Furthermore, molecular weights in the examples and the rest of the specification are weight average molecular weights, unless noted otherwise.

### EXAMPLE 1

The tissue microarray was purchased from LifeSpan Biosciences in Seattle. The specific array purchased was the multiple cancer array taken from multiple human sources.

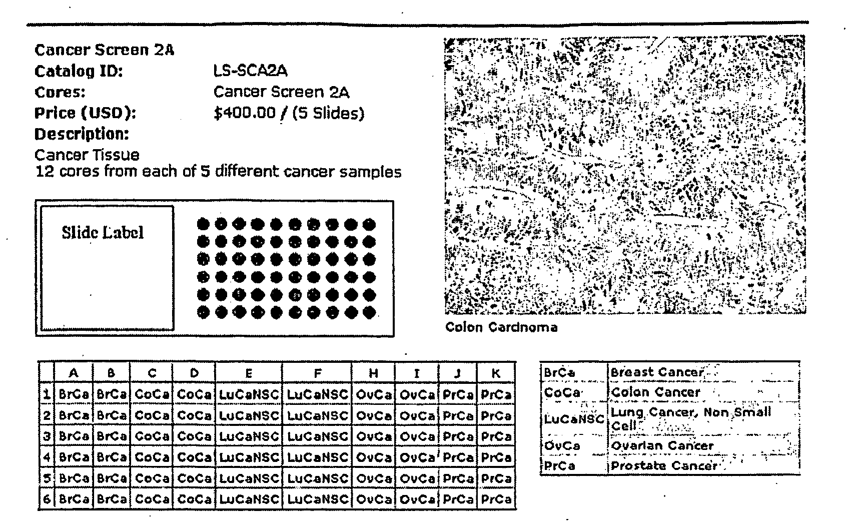

Five unstained sections were received. Two were stained with H&E, and two were used for mass spectrometry after antigen retrieval.

### Antigen retrieval method

1. Antigen retrieval is carried out using Target Retrieval Solution (product code S1699) available from DakoCytomation, Denmark: It is a modified citrate buffer, with a pH of 6.1 (modified with an unspecified amount of EDTA).
2. The procedure consists of taking 5µ tissue sections that have been previously formalin fixed and paraffin embedded and placed on charged (poly-L-lysine coated) slides to enhance adhesion.
3. The slides are heated to 60 degrees for 30-45 minutes. The sections are deparaffinized completely and rehydrated to deionized water by the following sequence.
   a. Prior to beginning the deparaffinization steps, begin to preheat the target retrieval solution in the Black & Decker Vegetable Steamer. Fill the base of the steamer with deionized water to the high fill line. Place the drip tray in the base and place a covered coplin jar of retrieval solution into the tray. Set the steamer timer for 75 minutes (which should result in a solution temperature of 95-99degC).
   b. The level of solution is provided to cover the tissues. Begin the next steps once the steamer timer has ~30 minutes remaining on the clock:
      i. Place warmed slides into xylene for 5 minutes. Repeat once.
      ii. Move slides to absolute ethanol for 3 minutes. Repeat once:
      iii. Move slides to 95% ethanol for 3 minutes. Repeat once.
      iv. Move slides to 70% ethanol for 3 minutes. Repeat once.
      v. Move to deionized water for 1 minute prior to placing slides into the preheated Target Retrieval Solution. The Target Retrieval Solution is purchased as a 10x concentrate that is diluted with deionized water prior to use. The coplin lid needs to be removed at this point for the lid to fit on the steamer.
      vi. The slides need to be immersed in the hot Target Retrieval Solution for 20 minutes.
      vii. Carefully remove the coplin jar from the steamer and place into the sink in order to run cold tap water around the coplin jar. Cool the slides with this running tap water for 20 minutes. Pour off the retrieval solution and rinse well with several changes of deionized water.
      viii. Place the slides into a room temperature wash buffer for 5 minutes prior to working with slides. (The buffer used currently is a Tris buffered saline solution containing Tween 20, pH7.6, available from DakoCytomation as a 10X concentrate. After dilution 1:10 with deionized water, the solution contains 50mM Tris-HCl, 150mM NaCl, 0.05% Tween 20 plus a preservative. The diluted wash buffer is stable for one week.)
      ix. The slides are now ready for analysis.

### Mass spectrometry on slides after antigen retrieval

Because the slides are too large for the MALDI device, the slides were hand cut to allow placement into a custom MALDI plate with a milled out recessed area. The histological slides were spotted with a trypsin solution and digested for 4.5 hours. After trypsin digestion the remaining surface liquid as well as the histological site of application was analyzed by MALDI-TOF: Additionally, a site was analyzed using MALDI without trypsinization.

### Procedure:

A positive control digest was performed using horse CytoChrome C. A 2.0 µg aliquot of trypsin was added to a sample containing 10.0 µg of Cytochrome C dissolved in **A**mmonium **Bi**-Carbonate (**ABC**) buffer. This sample was digested for 4.5 hours at 37°C.

A trypsin digest blank was also performed as a negative control. A 2.0 µg aliquot of trypsin was mixed with 100 µl of ABC buffer and digested in the same manner.

### Trypsin Tissue Digest Step

1) Prepared Stock 0.5 µg / µl trypsin with 100 mM NH4HCO3 pH 8.1 (**ABC**).
2) Added ~1.5ug trypsin per specified spot and digested for 4.5 hours at 37°C (Added 5 µl of additional ABC buffer every 20 minutes due to drying. At 1 hour samples were moved into a heated humidified chamber to alleviate the drying issue. The samples remained moist for the remainder of the digest time.)
3) Pipetted off the surface liquid from each spot and subsequently spotted onto the MALDI plate. Each tissue location was rinsed with 3 µl of milli-Q H20 which was subsequently spotted onto the correlating MALDI plate well.
4) Added 0.25 ul of alpha CHCA matrix to each tissue spot and to each sample on the MALDI plate.
5) Optimized MALDI settings using the pos/neg control digests and the "test" digest spot.
6) Analyzed the MALDI plate samples using reflectron mode. Also verified the sensitivity by analyzing with linear mode.
7) Continued to analyze the ON-tissue sample locations using linear mode and the MALDI plate samples using the Reflectron mode.

The prepared peptides were fingerprinted by using matrix assisted laser desorption ionization time-of-flight mass spectrometry (MALDI-TOF MS). Analyses were performed on examples 1-7 using a Voyager DE-STR (Applied Biosystems, Framingham, MA) in reflector and linear modes with positive ionization and an accelerating potential of either 20 or 25kV. The instrument was calibrated with peptide and protein standards from Sequazyme Kit (Applied Biosystems) for mass accuracy. The nitrogen laser has a wavelength of 337 ηm, and the instrument was set to acquire 150 spectra per sample spot. The laser beam has a linear spot diameter of approximately 150-200µm.

The results of the MALDI-TOF-MS are shown in Figure 1.

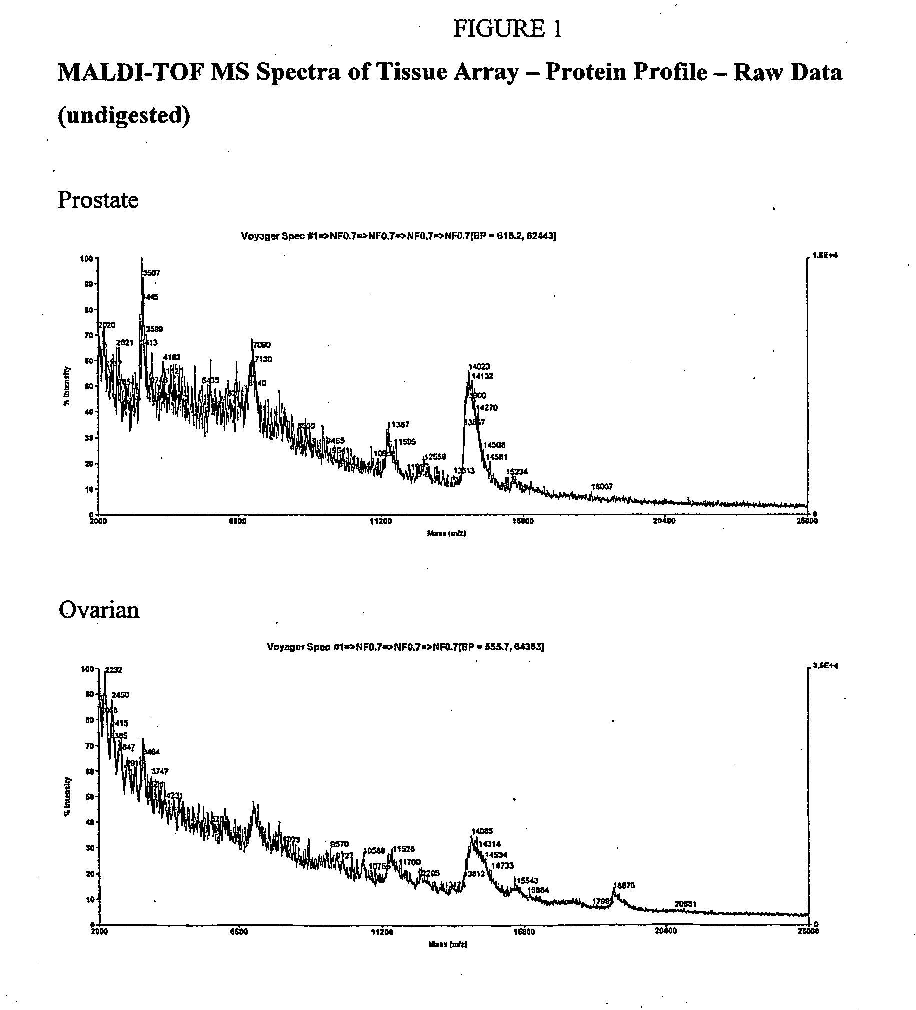

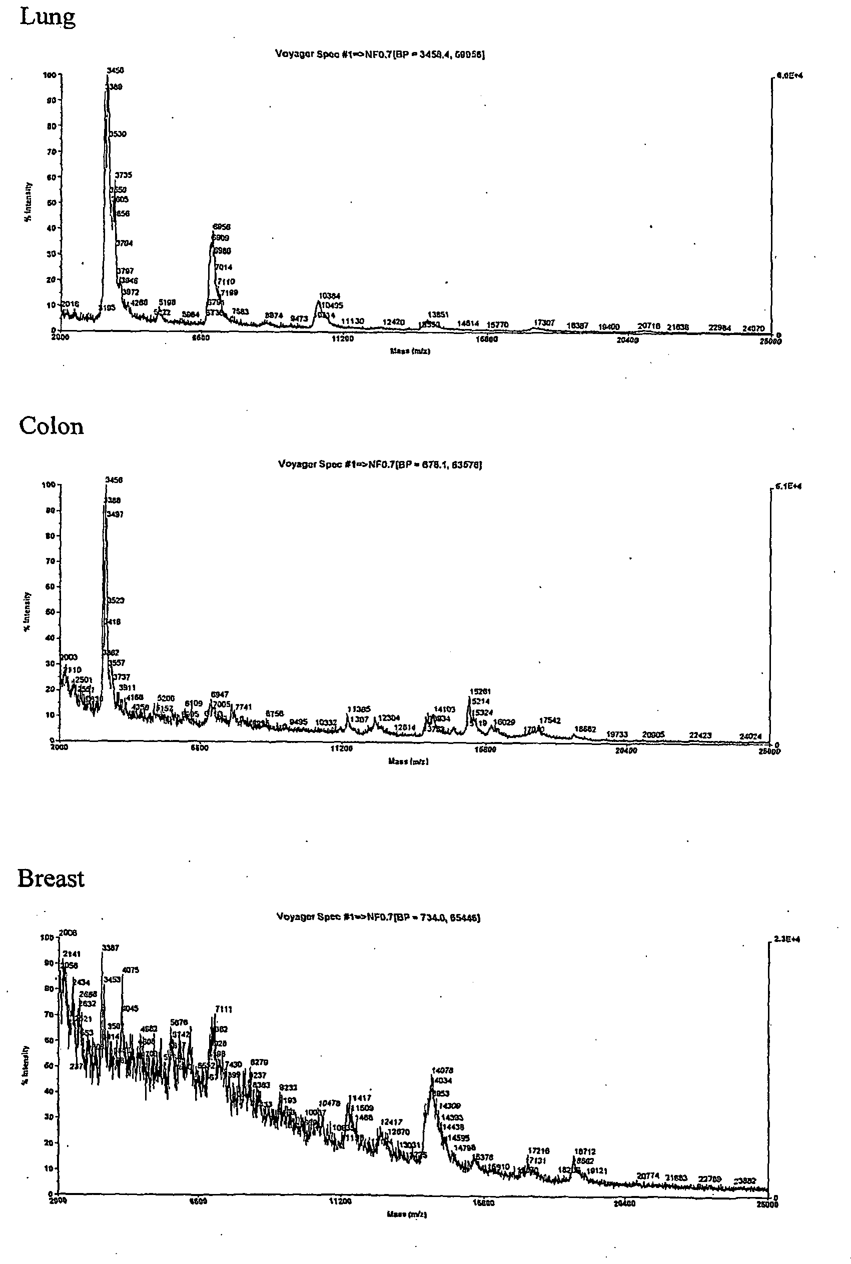

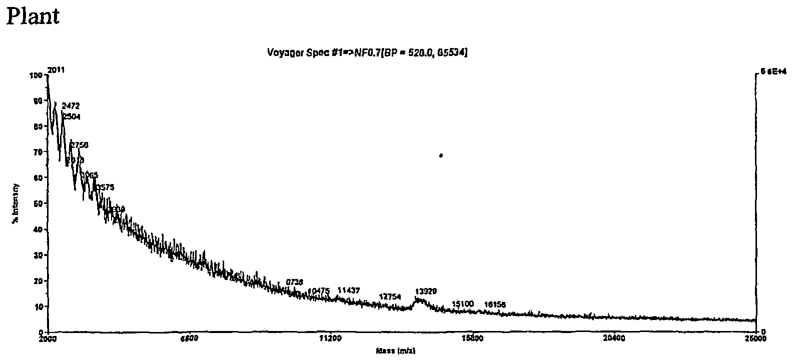

The data results are given as a file containing just under 100000 data points:

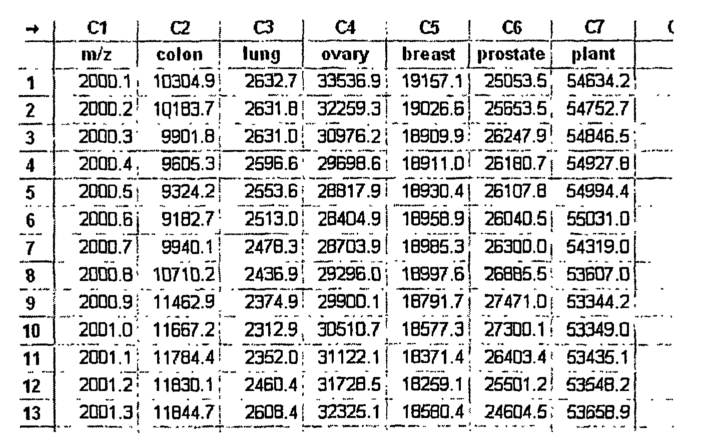

Because the experimental error is on the order of 2 daltons, and in order to reduce the dataset to a more manageable size, the data were indexed using a dummy variable.

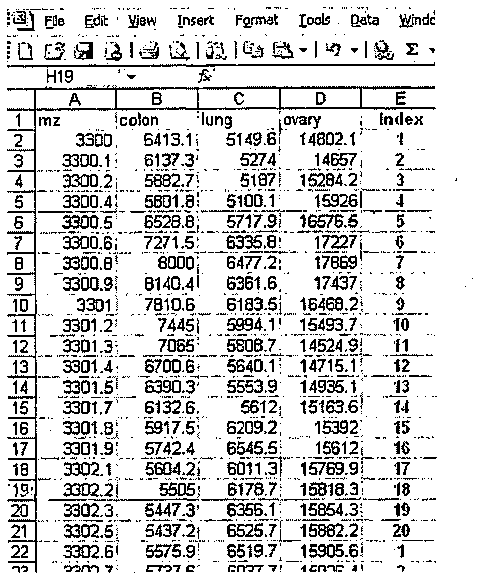

The entire dataset (6 x 99995 points) was sequenced using the index variable as a sorting parameter, resulting in a sequence of data that were now spaced by 2 daltons between each reading.

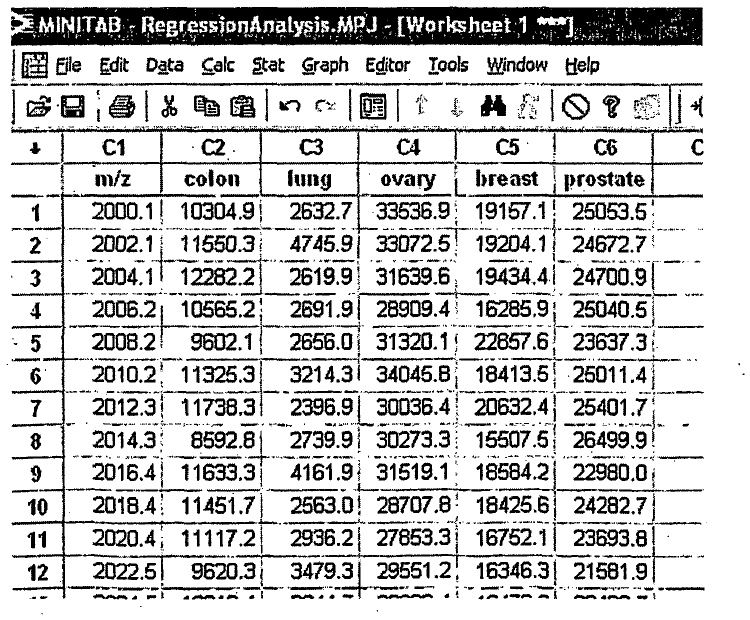

This "reduced" dataset, consisting of 4989 measurements per sample was used for subsequent analysis.

The data were normalized to the maximum and all measurements were converted to a fraction between 0 and 1 by dividing each value by the maximum of a given column.

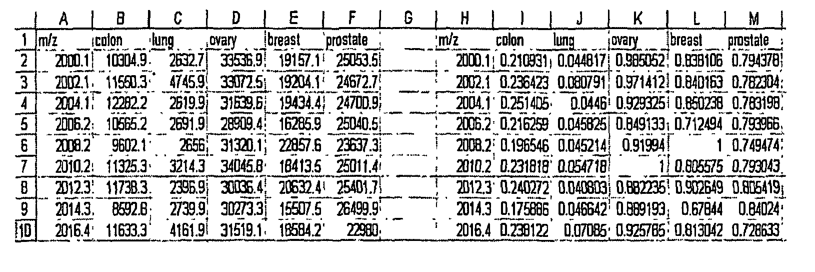

The normalized reduced data were then graphed to illustrate the relationship between relative intensities of observed peaks as a function of m/z ratio.

The relationship between relative intensity and m/z ratio illustrates multiple similar peaks:

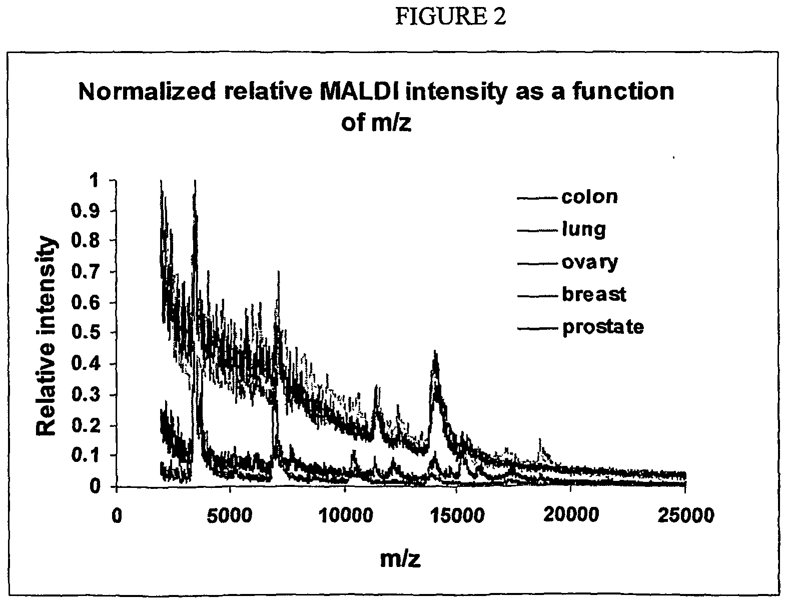

The undigested samples demonstrate a range of peaks, some of which are apparently overlapping. Additionally, as a general rule, the curves illustrate a monotonic decrease in intensity as a function of m/z value. This monotonic delay is modeled as a first order exponential decay.

The supporting data points of the analysis shown in Figure 2 were contained in an Excel spreadsheet:

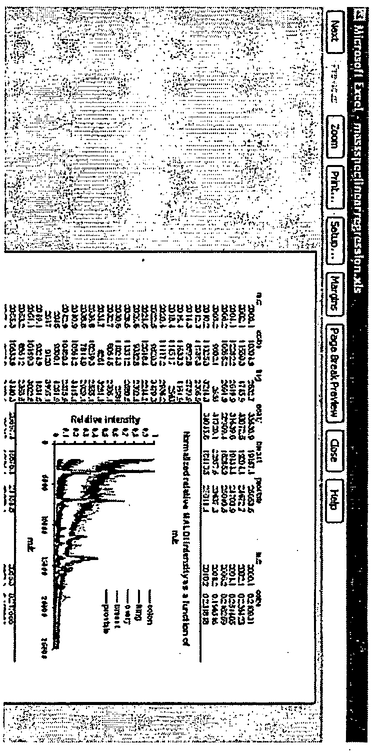

As a result of the chosen model, the data were plotted as log (measured intensity) as a function of m/z ratio. Below are two samples: colon, with a low background, and ovary, with a high background.

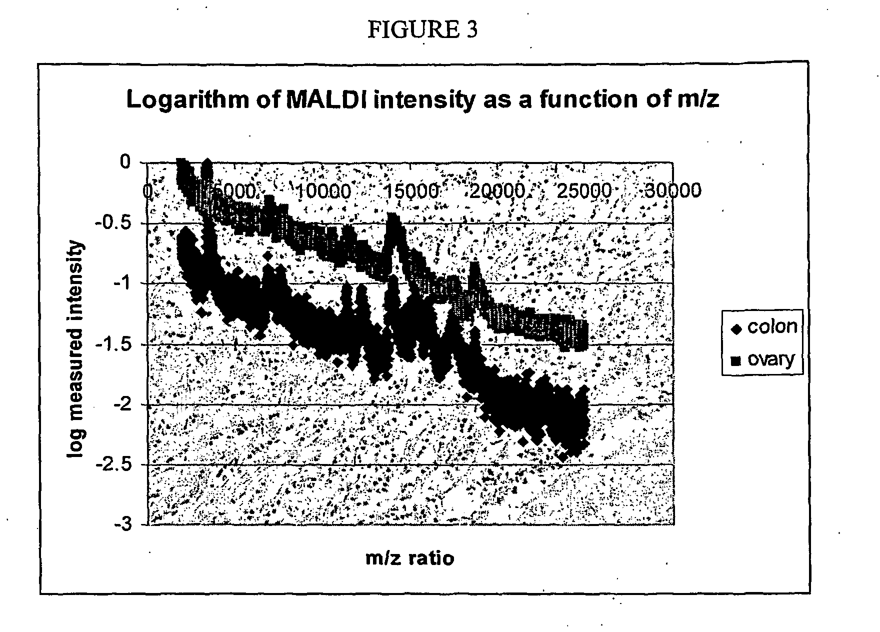

There is a strong linear relationship for each of the samples between measured intensity and m/z ratio. Linear regression analysis was performed for all five tumor samples. The error between measured and predicted was used to calculate a residual plot for each tumor sample.

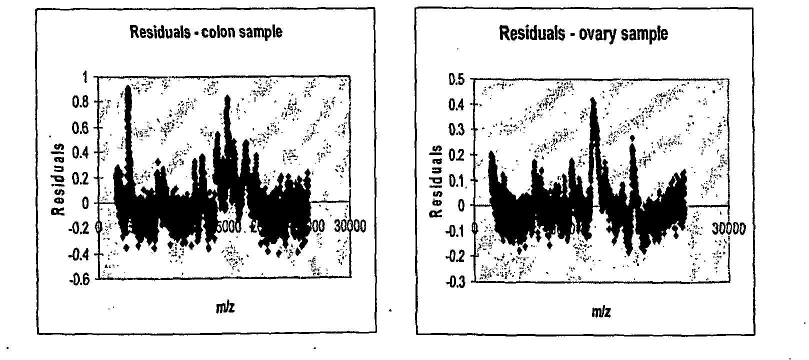

By subtracting the measured data from the derived linear equation, it is possible to filter out the effect of the baseline, illustrated by the residual plots for the data taken from the colon and ovary samples.

The normalized residuals illustrate both the shared and the unique signals from the tumor microarray samples.

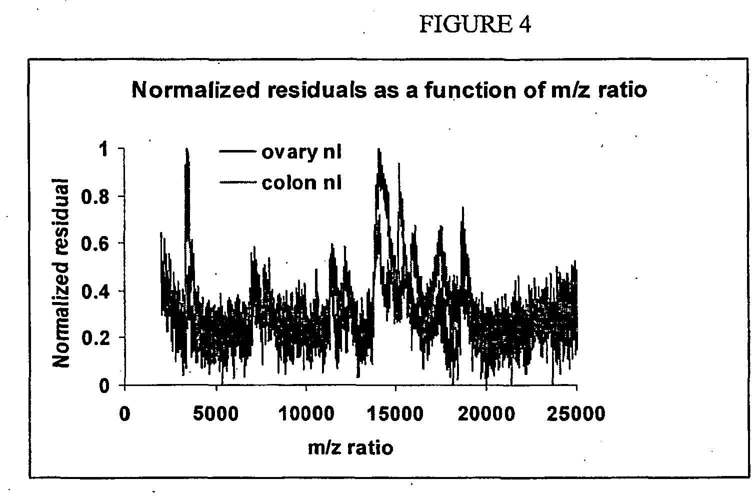

While the normalized residuals can distinguish between samples, it is not possible to identify the proteins using this method since peptide identification databases require digestion (most commonly with trypsin) and these results are obtained *without* modification. They are limited, therefore, to biomarkers without identification.

Trypsin treatment, followed by repeat analysis can allow for identification, if peaks observed before treatment are lost after treatment with trypsin, indicating that the protein of interest can now be identified using peptide databases.

### Histology step

Stained H&E sections were examined using a Leica microscope with PaxIt image analysis software. An image file with photomicrographs taken at 100x for each tumor has been created. Representative 100x photomicrographs of breast cancer samples A1.A1. and A5.E1 are given below

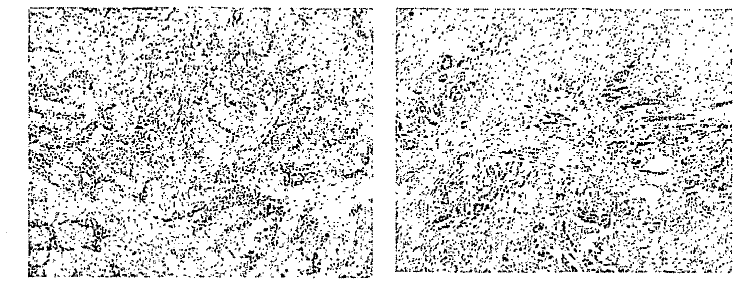

The images demonstrate one of the problems with cancer research: even though the two tumors are nominally identical, they do NOT have identical histologic patterns. This is typical of malignancies in general and breast carcinoma in particular.

Because there is an extensive literature dealing with prognosis of tumors, response to therapy of tumors, grade of tumors, etc based on histology; the question arose as to whether or not the mass spectra gave results that were unique, supplementary or identical to what could be obtained using standard histologic methods. As a first approximation, the histologic features within a square that would be similar (if not identical) to the area analyzed by the MALDI were analyzed.

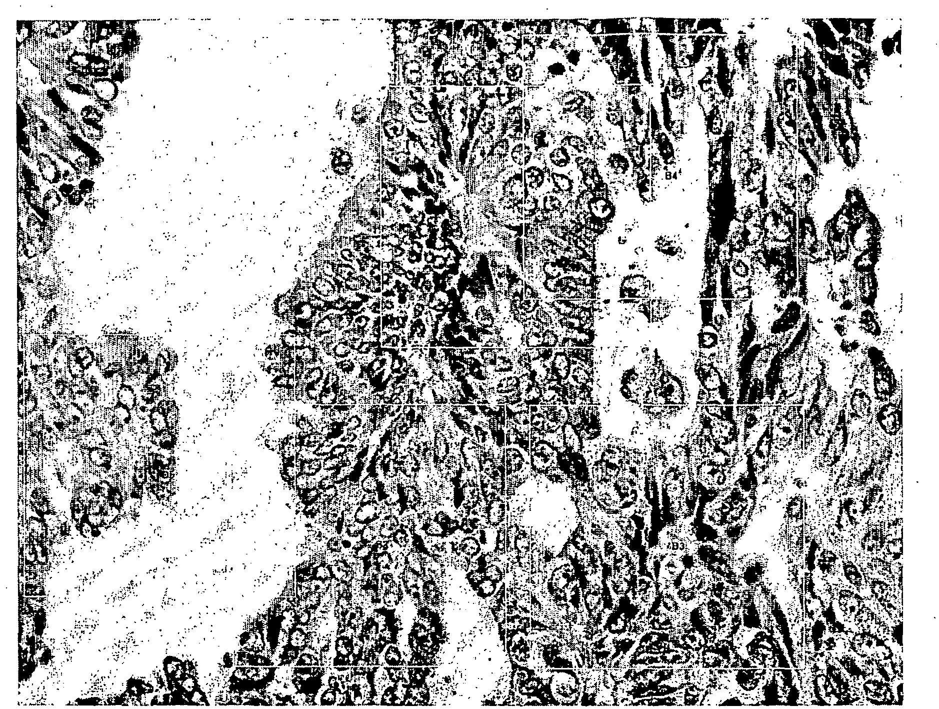

This image is taken from one of the ovarian carcinomas. Each box, outlined in yellow, is approximately the same area as the area analyzed by MALDI. At this magnification (400x) the predominant feature is the number of nuclei. The amount of stroma (collagen) is not a consideration. As a consequence, the differences among the various signals are NOT felt to reflect the difference in the body's response to the tumor (as reflected in its attempt to scar off the tumor) but rather these differences appear to be related to the nuclei.

In order to analyze if the MALDI analysis overlapped or duplicated in some way the analysis that could be done histologically, three tumors were chosen for analysis. The three tumors were chosen using the following method. Pairwise comparison of the mass spectral data without trypsinization was performed for all combinations of the 5 tumor specimens examined.

The scattergram for ovary and prostate demonstrated considerable similarity visually:

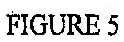

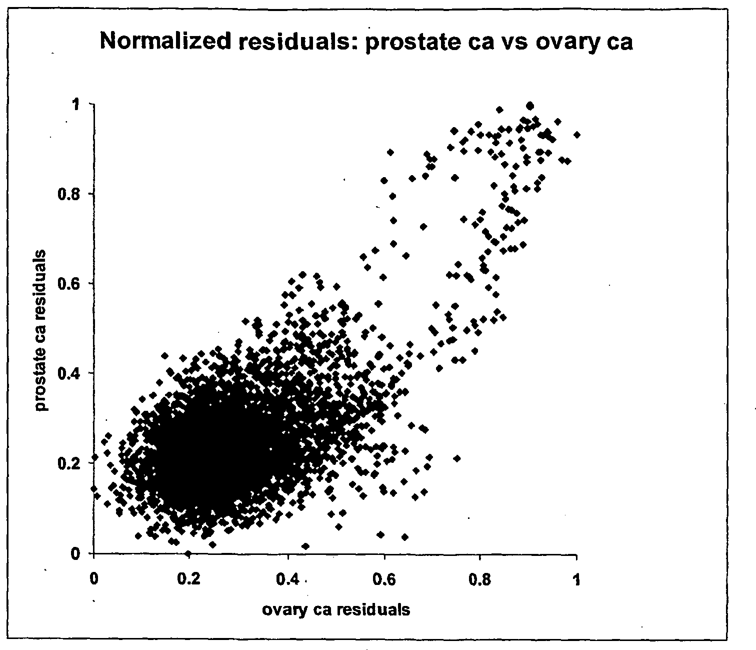

However, by comparison, the lung sample was distinctly different from ovary and/or prostate:

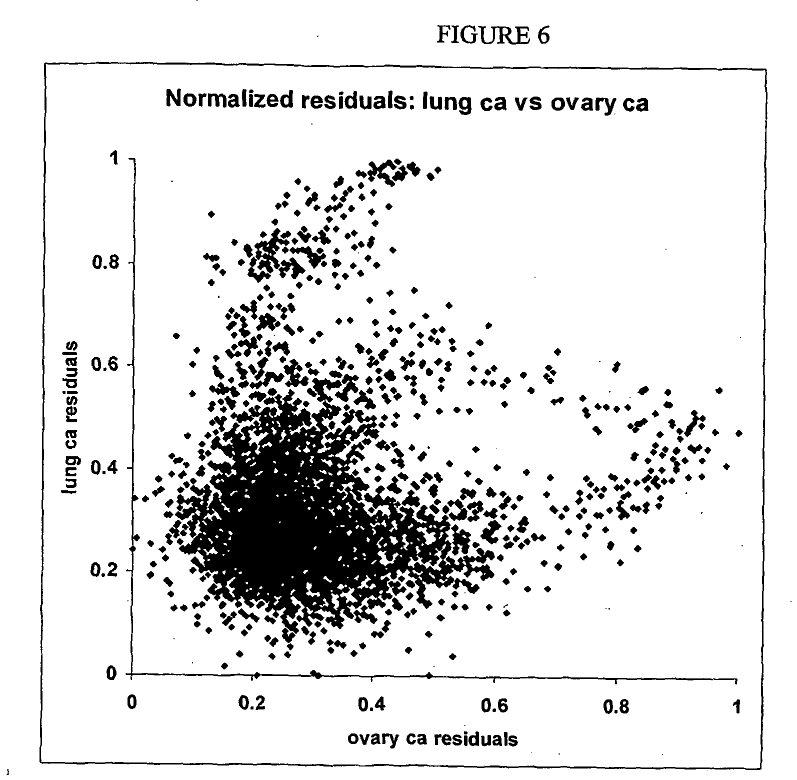

These three samples were chosen for histologic comparison to examine similar (prostate/ovary) and different (lung/ (prostate &ovary)) patterns.

For each of 5 areas, the nuclei were traced. The tracings yielded area and perimeter (sample demonstrated).
Prostate J5.E10 400x
A1 28.35168 25.9844
A2 20.95274 22.49948
A3 54.37891 36.04659
A4 67.53985 35.692
A5 41.51262 28.30794
A6 25.37246 24.7306
A7 48.02761 28.4942
A8 31.03625 22.69496
A9 59.48614 36.2408
A10 68.81666 37.64809

The equivalent radius was calculated using radius = 2xarea/perimeter (assuming circularity). The Radii were then plotted using a histogram for each:

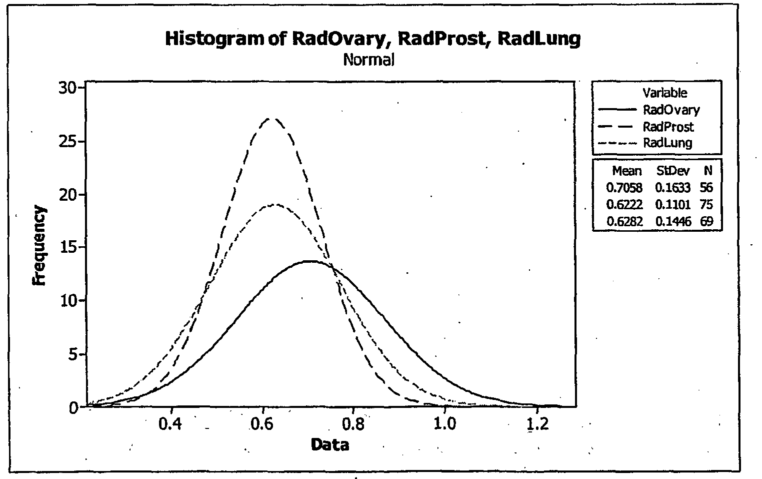

The number of nuclei varied from one sample to another but the histograms demonstrated that the ovarian nuclei had a higher mean and a broader range, but that the nuclei of the prostate sample and the lung sample were essentially identical. Because the MALDI curves are dramatically different but the nuclei are morphologically the same, the MALDI is giving a signal that represents events at a subnuclear or biochemical level and not simply duplicating the histology.

Various modifications and alterations to this invention will become apparent to those skilled in the art without departing from the scope of this invention. It should be understood that this invention is not intended to be unduly limited by the illustrative embodiments set forth herein and that such embodiments are presented by way of example only, with the scope of the invention intended to be limited only by the claims.

## Claims

1. A computerized method of analyzing an analyte, the method comprising:
providing a cellular sample comprising a chemically crosslinked analyte,
wherein the sample comprises a chemically fixed tissue section embedded in an organic solid material;
affixing the tissue section to a substrate:
reversing at least a portion of the chemical crosslinks in the crosslinked analyte to form decrosslinked anatyte after the tissue has been affixed to the substrate, wherein reversing at least a portion of the chemical crosslinks comprises cleaving the chemical crosslinks;
separating the cellular sample from the organic solid material;
generating a MALDI mass spectrum of at least a portion of the sample containing the decrosslinked analyte; and
analyzing the mass spectra using a digital computer, wherein the method of analyzing the mass spectra comprises:
a) storing in the digital computer a data set obtained from mass spectra from a plurality of cellular samples, wherein each sample is, or is to be assigned to a class within a class set comprising two or more classes, each class **characterized by** a different biological status, and wherein each mass spectrum comprises data representing signal strength as a function of time-of-flight, mass-to-charge ratio, or a value derived from time-of-flight or mass-to-charge ratio; and
b) forming a classification model which discriminates between the classes in the class set, wherein forming comprises analyzing the data set by executing code that embodies a classification process comprising a recursive partitioning process, which is a classification and regression tree process.

2. The method of claim 1 wherein the sample further comprises analytes that are not chemically crosslinked and analyzing comprises analysing both decrosslinked analyte and such analytes that were not chemically crosslinked.

3. The method of claim 1 further comprising separating the cellular sample from the solid organic material prior to reversing the crosslinking.

4. The method of claim 1 further comprising cleaving at least a portion of the bonds in the decrosslinked analyte to form analyte fragments; wherein generating the mass spectra of the decrosslinked analyte comprises generating the mass spectra of the analyte fragments.

5. The method of claim 1 wherein the data set is a known data set, and each sample is assigned to one of the classes before the data set is entered into the digital computer.

6. The method of claim 1 wherein the data set is formed by:
detecting signals in the mass spectra, each mass spectrum comprising data representing signal strength as a function of mass-to-charge ratio;
clustering the signals having similar mass-to-charge ratios into signal clusters;
selecting signal clusters having at least a predetermined number of signals with signal intensities above a predetermined value;
identifying the mass-to-charge ratios corresponding to the selected signal clusters; and
forming the data set using signal intensities at the identified mass-to-charge ratios.

7. The method of claim 1 wherein the cellular sample is a tissue microarray

8. The method of claim 1 wherein the cellular sample is a formalin-fixed, paraffin-embedded tissue.

9. The method of claim 1, further comprising in situ digestion.

10. The method of claim 9, wherein the in situ digestion comprises treating the decrosslinked analyte with an enzyme selected from the group consisting of typsin, chymotrypsin, pronase, and pepsin.

11. The method of claim 1, wherein the substrate is a glass slide.

## Patentansprüche

1. Computergesteuertes Verfahren zur Analyse eines Analyten, wobei das Verfahren Folgendes umfasst:
Bereitstellen einer zellulären Probe, die einen chemisch vernetzten Analyten umfasst, wobei die Probe einen in einem organischen festen Material eingebetteten chemisch fixierten Gewebeschnitt umfasst:
Befestigen des Gewebeschnitts an einem Substrat;
Rückgängigmachen von mindestens einem Teil der chemischen Vernetzungen in dem vernetzten Analyten, so dass man einen Analyten erhält, bei dem die Vernetzungen nicht mehr vorhanden sind, nachdem das Gewebe an dem Substrat befestigt wurde, wobei das Rückgängigmachen von mindestens einem Teil der chemischen Vernetzungen das Spalten der chemischen Vernetzungen umfasst,
Trennen der zellulären Probe von dem organischen festen Material;
Erzeugen eines MALDI-Massenspektrums von mindestens einem Teil der Probe, die den Analyten enthält, bei dem die Vernetzungen nicht mehr vorhanden sind; und
Analysieren der Massenspektren mit einem Digital-Computer, wobei das Analyse-Verfahren der Massenspektren Folgendes umfasst:
a) Speichern eines aus Massenspektren von einer Vielzahl zellulärer Proben erhaltenen Datensatzes in dem Digital-Computer, wobei jede Probe eine Klasse innerhalb einen Klassensatzes ist, der zwei oder mehrere Klassen umfasst, oder dieser zugeordnet wird, wobei jede Klasse durch einen unterschiedlichen biologischen Status gekennzeichnet ist, und wobei jedes Massenspektrum Daten umfasst, die die Signalstärke als Funktion der Laufzeit, Verhältnis von Masse zu Ladung oder als Wert veranschaulichen, der von der Laufzeit oder von dem Verhältnis von Masse zu Ladung hergeleitet ist; und
b) Bilden eines Klassifikationsmodells, das zwischen den Klassen in dem Klassensatz unterscheidet, wobei das Bilden das Analysieren des Datensatzes durch die Ausführung eines Codes umfasst, der ein Klassifizierungsverfahren umfasst, das ein rekursives Partitionierungsverfahren umfasst, bei dem es sich um ein Klassifikations- und Regressions-Baumverfahren handelt.

2. Verfahren nach Anspruch 1, wobei die Probe zudem nicht chemisch vernetzte Analyten umfasst, und das Analysieren das Analysieren des Analyten, bei dem die Vernetzungen nicht mehr vorhanden sind, und solcher Analyten, die nicht chemisch vernetzt wurden umfasst.

3. Verfahren nach Anspruch 1, zudem umfassend das Trennen der zellulären Probe von dem festen organischen Material vor dem Rückgängigmachen der Vernetzung.

4. Verfahren nach Anspruch 1, zudem umfassend das Spalten von mindestens einem Teil der Bindungen in dem Analyten, bei dem die Vernetzungen nicht mehr vorhanden sind, so dass Analytenfragmente erhalten werden; wobei das Erzeugen der Massenspektren des Analyten, bei dem die Vernetzungen nicht mehr vorhanden sind, das Erzeugen der Massenspektren der Analytenfragmente umfasst.

5. Verfahren nach Anspruch 1, wobei der Datensatz ein bekannter Datensatz ist, und jede Probe einer der Klassen zugeordnet wird, bevor der Datensatz in den Digital-Computer eingegeben wird.

6. Verfahren nach Anspruch 1, wobei der Datensatz gebildet wird durch:
Erfassen von Signalen in den Massenspektren, wobei jedes Massenspektrum Daten umfasst, die die Signalstärke als Funktion des Verhältnisses von Masse zu Ladung veranschaulichen;
Clustern der Signale mit ähnlichen Verhältnissen von Masse zu Ladung zu Signal-Clustern;
Auswählen von Signal-Clustern mit mindestens einer festgelegten Zahl von Signalen mit Signal-Intensitäten über einem festgelegten Wert;
Identifizieren der Verhältnisse von Masse zu Ladung entsprechend den ausgewählten Signal-Clustern; und
Bilden des Datensatzes mit Signalintensitäten bei den identifizierten Verhältnissen von Masse zu Ladung.

7. Verfahren nach Anspruch 1, wobei die zelluläre Probe ein Gewebe-Microarray ist.

8. Verfahren nach Anspruch 1, wobei die zelluläre Probe ein in Formalin fixiertes und in Paraffin eingebettetes Gewebe ist.

9. Verfahren nach Anspruch 1, zudem umfassend In situ-Spaltung.

10. Verfahren nach Anspruch 9, wobei die In situ-Spaltung das Behandeln des Analyten, bei dem die Vernetzungen nicht mehr vorhanden sind, mit einem Enzym, ausgewählt aus der Gruppe, bestehend aus Trypsin, Chymotrypsin, Pronase und Pepsin, umfasst.

11. Verfahren nach Anspruch 1, wobei das Substrat ein Glas-Objektträger ist.

## Revendications

1. Procédé informatisé d'analyse d'un analyte, le procédé comprenant :
la fourniture d'un échantillon cellulaire comprenant un analyte chimiquement réticulé, l'échantillon comprenant une coupe de tissu chimiquement fixée incluse dans un matériau solide organique ;
la fixation de la coupe de tissu à un substrat ;
l'inversion d'au moins une partie des réticulations chimiques dans l'analyte réticulé pour former un analyte déréticulé après que le tissu a été fixé au substrat,
où l'inversion d'au moins une partie des réticulations chimiques comprend le clivage des réticulations chimiques ;
la séparation de l'échantillon cellulaire du matériau solide organique ;
la génération d'un spectre de masse MALDI d'au moins une partie de l'échantillon contenant l'analyte déréticulé ; et
l'analyse des spectres de masse en utilisant un ordinateur numérique, le procédé d'analyse des spectres de masse comprenant :
a) le stockage dans l'ordinateur numérique d'un jeu de données obtenu à partir de spectres de masse d'une pluralité d'échantillons cellulaires, où chaque échantillon est, ou doit être affecté à une classe dans un jeu de classes comprenant deux classes ou plus, chaque classe étant **caractérisée par** un statut biologique différent, et chaque spectre de masse comprenant des données représentant l'intensité de signal en fonction du temps de vol, du rapport masse/charge, ou une valeur dérivée du temps de vol ou du rapport masse/charge ; et
b) la formation d'un modèle de classification qui distingue les classes dans le jeu de classes, la formation comprenant l'analyse du jeu de données en exécutant un code qui effectue un processus de classification comprenant un procédé de partitionnement récursif, qui est une classification et un procédé d'arborescence de régression.

2. Procédé de la revendication 1 dans lequel l'échantillon comprend en outre des analytes qui ne sont pas chimiquement réticulés et l'analyse comprend l'analyse à la fois de l'analyte déréticulé et des analytes qui n'ont pas été chimiquement réticulés.

3. Procédé de la revendication 1 comprenant en outre la séparation de l'échantillon cellulaire du matériau organique solide avant l'inversion de la réticulation.

4. Procédé de la revendication 1 comprenant en outre le clivage d'au moins une partie des liaisons dans l'analyte déréticulé pour former des fragments d'analyte ; la génération des spectres de masse de l'analyte déréticulé comprenant la génération des spectres de masse des fragments d'analyte.

5. Procédé de la revendication 1 dans lequel le jeu de données est un jeu de données connu, et chaque échantillon est affecté à une des classes avant que le jeu de données ne soit entré dans l'ordinateur numérique.

6. Procédé de la revendication 1 dans lequel le jeu de données est formé par :
détection de signaux dans les spectres de masse, chaque spectre de masse comprenant des données représentant l'intensité de signal en fonction du rapport masse/charge ;
groupage des signaux ayant des rapports masse/charge similaires dans des groupes de signaux ;
sélection de groupes de signaux ayant au moins un nombre prédéterminé de signaux avec des intensités de signal au-dessus d'une valeur prédéterminée ;
identification des rapports charge/masse correspondant aux groupes de signaux sélectionnés ; et
formation du jeu de données en utilisant des intensités de signal aux rapports masse/charge identifiés.

7. Procédé de la revendication 1 dans lequel l'échantillon cellulaire est une micropuce de tissu.

8. Procédé de la revendication 1 dans lequel l'échantillon cellulaire est un tissu fixé par le formol, inclus dans la paraffine.

9. Procédé de la revendication 1 comprenant en outre une digestion in situ.

10. Procédé de la revendication 9 dans lequel la digestion in situ comprend le traitement de l'analyte déréticulé avec une enzyme choisie dans le groupe constitué de la trypsine, la chymotrypsine, la pronase et la pepsine.

11. Procédé de la revendication 1 dans lequel le substrat est une lame de verre.
